(19) Europäisches Patentamt
European Patent Office
Office européen des brevets

(11) EP 4 512 392 A2

# (12) EUROPEAN PATENT APPLICATION

(43) Date of publication:
**26.02.2025 Bulletin 2025/09**

(21) Application number: **24192299.6**

(22) Date of filing: **30.11.2021**

(51) International Patent Classification (IPC):
***A61K 9/20*** *(2006.01)*

(52) Cooperative Patent Classification (CPC):
(C-Sets available)
**A61K 9/209; A61K 31/18; A61K 31/4725;**
A61K 9/2009; A61K 9/2031 (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.12.2020 EP 20211022**

(62) Document number(s) of the earlier application(s) in accordance with Art. 76 EPC:
**21819877.8 / 4 255 398**

(71) Applicants:
- **Adalvo Limited**
  **San Gwann, SGN 3000 (MT)**
- **Adamed Pharma S.A.**
  **05-152 Czosnow (PL)**

(72) Inventor: **BOLTROMIUK, Tomasz**
**02-496 Warszawa (PL)**

(74) Representative: **Vossius & Partner**
**Patentanwälte Rechtsanwälte mbB**
**Siebertstrasse 3**
**81675 München (DE)**

Remarks:
•This application was filed on 01-08-2024 as a divisional application to the application mentioned under INID code 62.
•Claims filed after the date of filing of the application (Rule 68(4) EPC)/after the date of receipt of the divisional application

## (54) ORALLY-ADMINISTERED PREPARATION CONTAINING SOLIFENACIN AND TAMSULOSIN

(57) the present invention relates to a bilayer tablet for oral administration comprising (1) a first modified release layer comprising tamsulosin or a pharmaceutically acceptable salt thereof and a matrix forming polymer and (2) a second immediate release layer comprising solifenacin or a pharmaceutically acceptable salt thereof and at least one water-insoluble diluent, wherein the tablet is optionally film coated.

EP 4 512 392 A2

(52) Cooperative Patent Classification (CPC): (Cont.)

C-Sets
**A61K 31/18, A61K 2300/00;**
**A61K 31/4725, A61K 2300/00**

## Description

### Field of the invention

The present invention relates to a solid pharmaceutical composition for oral administration comprising solifenacin and tamsulosin.

More specifically the present invention relates to a tablet for oral administration comprising (1) a first modified release layer comprising tamsulosin or a pharmaceutically acceptable salt thereof and a matrix forming polymer and (2) a second immediate release layer comprising solifenacin or a pharmaceutically acceptable salt thereof, in particular solifenacin succinate, and at least one water-insoluble diluent, wherein the tablet is optionally film coated, preferably in the form of a bilayer tablet.

### Background art

Tamsulosin is the INN of 5-[(2R)-2-[[-ethoxyphenoxy)ethyl]amino]propy]-2-methoxybenzenesulfonamide and has the following structural formula (I):

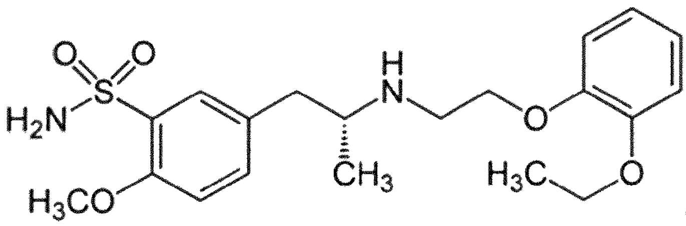

(I)

Tamsulosin is known as an $\alpha$1-adrenergic receptor antagonist. For many years, tamsulosin or a salt thereof, in particular its hydrochloride salt, has been widely used in the treatment of dysuria associated benign prostatic hyperplasia (BPH) by reducing prostatic pressure in urethral pressure profile. A number of medicaments comprising tamsulosin hydrochloride for the treatment of lower urinary tract symptoms, such as symptomatic benign prostatic hyperplasia (BPH), chronic prostatitis, and to facilitate the passage of kidney stones are currently on the market in Europe and the USA. The dosage strength of tamsulosin hydrochloride tablets for oral administration is generally 0.4 mg per single unit dose (prolonged release).

Pharmaceutical formulations of tamsulosin are available in the form of a modified-release (MR) capsule formulation or an orally-controlled absorption system (OCAS®) tablet formulation. Controlled release tablet formulations making use of the oral-controlled absorption system (OCAS®) have been introduced onto the European market under the name of Omnic OCAS® and Mapelor OCAS®

The OCAS® tablet formulation was designed to overcome the low absorption of tamsulosin from the colon resulting in a more constant 24-hour plasma concentration of tamsulosin without food effect. For example Yamanouchi Pharmaceutical Co. Ltd's patent application EP 661 045 describes sustained release hydrogel preparations comprising (1) at least one drug such as tamsulosin HCl, (2) a hydrophilic additive or hydrophilic base, such as polyethylene oxide, ensuring penetration of water into the preparation and (3) a hydrogel-forming polymer such as polyethylene glycol.

EP 1 523 994 by Astellas relates to the product Omnic OCAS® and discloses a pharmaceutical composition for controlled release comprising a sized product, which comprises tamsulosin, polyethylene oxide with a viscosity-average molecular weight of 2,000,000 or more and a size controlling agent. The size controlling agent is one or two or more selected from the group consisting of polyethylene glycol that is solid at room temperature, hydroxypropylmethyl cellulose of 2 to 15 mPas (2%w/v), hydroxypropyl cellulose of 2 to 10 mPas (2% w/v), and methyl cellulose of 2 to 15 mPas (2% w/v).

Accordingly, the currently marketed tamsulosin tablet formulation Omnic OCAS® comprises a high molecular weight polyethylene oxide as the hydrogel-forming polymer (Macrogol 7,000,000) and polyethylene glycol as hydrophilic base (Macrogol 8,000).

Solifenacin is the INN of(3R)-1-Azabicyclo[2.2.2]octan-3-yl (1S)-1-phenyl-3,4-dihydroisoquinoline-2(1*H*)-carboxylate, a muscarinic receptor antagonist with the following structural formula (II):

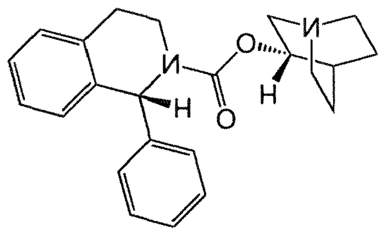

(II).

Solifenacin, a competitive muscarinic acetylcholine receptor antagonist, is used as a urinary spasmodic for the treatment of overactive bladder, alleviating incontinence, urinary frequency, and urinary urgency. Dosage strengths of solifenacin succinate tablets for oral administration are 5 and 10 mg per single unit dose. Solifenacin is marketed under the tradename of Vesicare® as a film coated tablet. Pharmaceutical compositions comprising solifenacin are known inter alia from EP 1 832 288, EP 1 852 117 or EP 2 178 507. EP 2 146 693 discloses stable formulations of amorphous solifenacin succinate.

Because the combination therapy using tamsulosin and solifenacin or their pharmaceutically acceptable salts has shown to be clinically effective, providing additional benefits in the treatment of lower urinary tract symptoms associated with benign prostatic hyperplasia, it is desirable to provide a combined formulation of the two active agents combining both in a single formulation thereby improving i.a. patient compliance.

Combination formulations combining a modified release component comprising tamsulosin with an immediate release component comprising solifenacin have been proposed in the art.

As the desired optimized drug dissolution profiles for the individual active agents to be achieved differ fundamentally, it is of utmost importance that the desired release profile for each of the active agents is not compromised when formulated in a combination formulation. Thus as set out in EP 2 394 648, a combination product prepared by simply combining a modified release portion of tamsulosin formulated corresponding to the marketed product Omnic OCAS® with an immediate release portion of solifenacin formulated corresponding to the marketed product Vesicare® results in tablets with an altered dissolution profile and a decreased maximum release for solifenacin, which is not bioequivalent to the combined use of the mono preparations. Thus, it was found out that the dissolution of solifenacin was decreased and was below 85% at 30 minutes and the maximum dissolution rate of solifenacin was less than 90%.

EP 2 394 648 by Astellas, relating to the combination formulation currently marketed as Vesomni® discloses a tablet comprising a modified release and an immediate release layer which combines a first modified release layer comprising tamsulosin, a hydrogel forming polymer and a hydrophilic base and a second immediate release layer comprising solifenacin and at least one hydrophilic substance from the group consisting of D-mannitol, maltose, polyethylene glycol and polyvinylpyrrolidone in a single formulation. However, it has been found that the release of solifenacin from the marketed formulation according to EP 2 394 648 (Vesomni®) is still adversely affected by the presence of the modified release layer comprising tamsulosin. The dissolution of solifenacin from Vesomni® appears to be reduced when compared to the single formulation. Thus, the dissolution was found to be about 90% at 30 minutes and the maximum dissolution of solifenacin was about 95%, indicating that the dissolution of solifenacin from the immediate layer is still diminished by the influence of the hydrogel based modified release layer. It is understood that the marketed formulation Vesomni® is only available as Vesomni® 6 mg/0.4 mg modified-release tablets. This product is also simply referred to as Vesomni® in the context of the present application.

The tablet formulation according to EP 2 394 648 comprises mannitol in the immediate release layer. It has been found that the presence of mannitol, however, may be problematic in terms of tablet compression as only low hardness values for the tablets may be obtained. Moreover, further processing problems may arise by virtue of the fact that the brittle solifenacin layer comprising mannitol as an excipient is difficult to combine with the elastic tamsulosin layer.

Also, it has been shown that large amounts of mannitol should be avoided in some patient groups as mannitol negatively influences peristaltic of the intestines and may i.a. cause diarrhoea.

A further combination formulation comprising a modified release layer comprising tamsulosin and an immediate release part comprising solifenacin is disclosed in EP 3 697 392 by Synthon. The formulation suggested in EP 3 697 392 comprises a water insoluble diluent in the immediate release part, such as Avicel DG (co-processed microcrystalline cellulose and calcium phosphate), silicon dioxide is then added as a glidant. It has, however, been found that here too problems arise with regard to compression to bilayer tablets. Thus, such tablets have been shown to have a low mass and content uniformity (uniformity of mass of single dose criteria was not met), surface defects (capping) and high friability during film coating as well an increase in solifenacin impurities. These problems appear to be primarily due to poor flowability of the tableting powder for the immediate release portion and problems arising from the need of combining of two layers with different plasticity/elastic behavior/ deformation upon compression.

There is thus still the need for an improved tablet for oral administration comprising a modified release layer comprising tamsulosin or a pharmaceutically acceptable salt thereof and an immediate release layer comprising

solifenacin succinate and a process for preparing the same, overcoming the problems encountered with the prior art formulations, such as reduced dissolution of solifenacin from the immediate release rate and poor quality of the bilayer tablet, such as tablet weight variation and weight variation of the individual layers by poor flow characteristics of the tablet materials and low hardness due to capping or non-compressibility, as discussed above.

## Brief description of the invention

The present invention provides a tablet for oral administration comprising:

- a modified release layer comprising tamsulosin or a pharmaceutically acceptable salt thereof, a matrix forming polymer, and optionally one or more excipients selected from a hydrophilic additive, diluent, glidant and lubricant;
- an immediate release layer comprising:

    solifenacin succinate, in particular crystalline solifenacin succinate, and a water-insoluble diluent, wherein the water insoluble diluent is selected from a water-insoluble excipient with brittle deformation during compression and a water-insoluble excipient with plastic deformation during compression or combinations thereof,
    wherein the tablet is optionally film coated.
    Moreover, the invention provides a process for preparing the tablet according to the present invention. The tablet according to the present invention may be used in the treatment of moderate to severe storage symptoms such as urgency and increased micturition frequency and voiding symptoms associated with benign prostatic hyperplasia (BPH).

## Detailed description of the invention

The invention provides an improved solid combination formulation of solifenacin and tamsulosin, having the same dissolution profile for solifenacin from the immediate release layer as the mono preparation by providing a tablet formulation for oral administration, wherein the immediate release layer is based on a water-insoluble diluent, wherein the water insoluble diluent is selected from a water-insoluble excipient with brittle deformation during compression and a water-insoluble excipient with plastic deformation during compression or combinations thereof. Moreover, the tablet according to the present invention also has the same dissolution profile for tamsulosin from the modified release layer as the mono preparation.

Water-insoluble diluents or fillers do not interact with the hydrogel layer used for obtaining the modified release profile for tamsulosin, thereby achieving next to 100% release from/dissolution of the immediate release layer.

More specifically the present invention provides

1. A tablet for oral administration comprising

    (1) a first modified release layer comprising tamsulosin or a pharmaceutically acceptable salt thereof, a matrix forming polymer and optionally one or more excipients selected from a hydrophilic additive, a diluent, a glidant and a lubricant;
    and
    (2) a second immediate release layer comprising crystalline solifenacin succinate and a water-insoluble excipient with brittle deformation during compression, selected from dicalcium phosphate (DCP) and dicalcium phosphate anhydrous (DCPA) and a water-insoluble excipient with elastic deformation during compression which is silicified microcrystalline cellulose (SMCC) or combinations thereof, and optionally one or more excipient selected from a disintegrant, a binder and a lubricant.

In particular the second immediate release layer comprises crystalline solifenacin succinate and a combination of silicified microcrystalline cellulose (SMCC) and dicalcium phosphate (DCP) or anhydrous dicalcium phosphate (DCPA) at a ratio of 2.0-3.8 : 0.7-1.3, preferably at a ratio of 3:1, and optionally one or more excipient selected from a disintegrant, binder and lubricant, wherein the tablet is optionally film coated.

By virtue of the fact that SMCC is used, $SiO_2$ is incorporated as an integral, non-separable part of the filler/diluent, the preparation of the bilayer formulation is significantly facilitated and no addition of extra glidant is necessary. The tablets of the present invention thus exhibit excellent mass and content uniformity, no surface defects and low friability during film coating. Moreover, even upon long term storage, the tablets are stable and no increase in solifenacin impurities is observed.

Components of formulations according to the present invention:

According to the classification provided by Rowe R.J. and Roberts R.C., materials with a yield pressure ($P_y$) of below < 80 MPa are defined as soft/plastic, whereas above this limit, excipients are expected to exhibit a more hard/brittle behaviour (Pharmaceutical Powder Compaction Technology: Editors: Goran Alderborn and Chris Nystrom. Marcel Dekker, New York, NY, 1996)

The yield pressure ($P_y$) can readily be determined from the Heckel equation (Heckel RW (1961) Density-Pressure Relationship in Powder Compaction. Trans Met Soc AIME 22: 671-675):

$$\ln(1/1-D)=K\bullet P+A \quad (1)$$

where D is the relative density of the compact, P is the applied pressure, A is a constant suggested to represent particle rearrangement and the reciprocal of K is used to calculate apparent mean yield pressure (Py), by conventional methods such as disclosed in S. Patel et al., International Journal of Pharmaceutics 389 (2010) 66-73.

According to the present invention, water-insoluble excipient, with plastic deformation during compression is silicified microcrystalline cellulose (SMCC), i.e. co-processed $SiO_2$ with microcrystalline cellulose (MCC), an agglomerated composite from MCC (Ph.Eur.) and colloidal silicon dioxide (Ph.Eur.). The use of high density silicified MCC (98% MCC +2% $SiO_2$), with a mean particle size of 125 $\mu$m (determined by laser diffraction) and a BET of about 6$m^2$/g and a bulk density of 0.25-0.37 or 0.38-0.50 g/ml such as qualities commercially available as Prolsolv 90 or Prosolv HD 90 is particularly preferred, wherein qualities with a mean particle size of 125 $\mu$m (determined by laser diffraction) and a specific surface area (BET) of about 6$m^2$/g and a bulk density of 0.38-0.50 available as Prosolv 90 HD are most preferred in all embodiments of the invention.

According to the present invention, the water-insoluble excipient with brittle deformation during compression is dibasic calcium phosphate (DCP) or anhydrous dibasic calcium phosphate (DPA) with a specific surface area of 20$m^2$/g to 60$m^2$/g, more preferably greater than 35 $m^2$/g, most preferably about 40$m^2$/g as described in US 5,486, 365, spherically shaped with a mean particle size 115 $\mu$m to about 120 $\mu$m. The use of anhydrous dibasic calcium phosphate (DCPA) with a mean particle size of 120$\mu$m and BET>35$m^2$/g, preferably 40$m^2$/g, with a mean particle size of 120 $\mu$m as e.g. commercially available as Fujicalin is particularly preferred in all embodiments of the invention.

The specific surface area (BET) may readily be measured by conventional methods such as set out in the European Pharmacopeia 5.1, section 2.9.26 Specific Surface Area by Gas Adsorption.

The bulk density may readily be measured by conventional methods such as set out in the European Pharmacopeia 7.0, section 2.9.34 Bulk Density and Tapped Density of Powders.

The particle size may readily be measured by conventional methods such as set out in the European Pharmacopeia 8.0, section 2.9.31 Particle Size Analysis by Laser Light Diffraction.

According to the present invention the disintegrant in the immediate release layer may be selected from crospovidone, alginate salts, cellulose derivatives, croscarmellose salts, more preferably form L-HPC (binder/disintegrant) and croscarmellose sodium. Most preferably the binder/disintegrant of the immediate release layer is L-HPC.

According to the present invention the lubricant in both layers can be selected from calcium stearate, glycerin monostearate, glyceryl behenate, glyceryl palmitostearate, hydrogenated castor oil, light mineral oil, magnesium lauryl sulfate, magnesium stearate, medium chain glycerides, mineral oil, myristic acid, palmitic acid, poloxamer, polyethylene glycol, sodium lauryl fumarate, stearic acid, talc and zinc stearate. Magnesium stearate is most preferred as a lubricant in both layers.

According to the present invention the matrix forming polymer of the modified release layer is Macrogol 5 000 000 to Macrogol 9 000 000, preferably Macrogol 7 000 000 (PEO 7 000 000).

The hydrophilic additive in the modified release layer is preferably Macrogol 8 000 (PEG 8 000).

However, in a preferred embodiment the modified release layer does not comprise a hydrophilic additive.

The diluent in the modified release layer may be selected from the group consisting of lactose, mannitol, microcrystalline cellulose, dicalcium phosphate, pregelatinized starch, HPMC, HPC. Preferred are non-saccharide excipients such as microcrystalline cellulose (MCC), dicalcium phosphate (DCP) and pregelatinized starch.

The use of MCC type 200 is particularly preferred.

The glidant in the modified release layer may be selected from tribasic calcium phosphate, cellulose powder, colloidal silicon dioxide, hydrophobic colloidal silica, magnesium oxide, magnesium silicate, magnesium trisilicate, silicon dioxide, and talc. The use of anhydrous colloidal silicon dioxide as a glidant in the modified release layer is preferred.

A film coat comprising HPMC, Macrogol 6000 and a (dye) coat may optionally applied on the tablet core, such as Colorcon Opadry®. Preferred is the use of Opadry® complete film coating system 03F45072 with water as the solvent.

According to the invention alcohols or water or mixtures thereof may be used as the granulation solvent, the use of ethanol water mixtures, in particular of ethanol 96 %(v/v) with water at a ratio of 70:30 is preferred.

The use of combinations of water-insoluble excipients is preferred, wherein a combination of silicified microcrystalline cellulose (SMCC) and dicalcium phosphate (DCP) or anhydrous dicalcium phosphate (DCPA) at a ratio of 2.0-3.8 : 0.7-1.3 are preferred, a ratio of 3:1 is particularly preferred.

In a preferred embodiment the tablets according to the present invention comprising a first modified release layer and a second immediate release layer are in form of a bi-layered tablet comprising two parallel distinct layers.

According to the present invention an inclusion of an additional intermediate layer of inert material may to ensure the layers do not make contact is not necessary.

Any commonly used type of bilayer tablet press may be used to prepare the tablet of the present invention.

Tamsulosin

As used herein "tamsulosin" refers to tamsulosin or a pharmaceutically acceptable salt thereof unless stated otherwise.

Tamsulosin forms pharmaceutically acceptable salts with various inorganic and organic acids. These pharmaceutically acceptable salts may be used in accordance with the present invention. Examples of the salts include salts with inorganic acids such as hydrochloric acid, sulfuric acid, and phosphoric acid; salts with organic acids such as fumaric acid, malic acid, citric acid, and succinic acid; salts with alkali metals such as sodium and potassium; and salts with alkali earth metals such as calcium and magnesium. Tamsulosin hydrochloride may be used in the preferred embodiment of the present invention.

The dose of tamsulosin or a pharmaceutically acceptable salt thereof may be appropriately determined for each patient in accordance with, for example, the route of administration, symptoms of a disease, the age and the sex of a patient to be treated, or the like. When tamsulosin hydrochloride is orally administered to an adult, the daily dose is approximately 0.1 mg to 1.6 mg as the active ingredient and is orally administered once a day.

Preferably the unit dose is 0.4 mg.

Solifenacin

As used herein "solifenacin" refers to solifenacin or a pharmaceutically acceptable salt thereof, unless stated otherwise.

Solifenacin forms pharmaceutically acceptable salts with various inorganic and organic acids. These pharmaceutically acceptable salts may be used in accordance with the present invention. Examples of the salts include acid addition salts with mineral acids such as hydrochloric acid, hydrobromic acid, hydriodic acid, sulfuric acid, nitric acid, and phosphoric acid; and acid addition salts with organic acids such as formic acid, acetic acid, propionic acid, oxalic acid, malonic acid, succinic acid, fumaric acid, maleic acid, lactic acid, malic acid, citric acid, tartaric acid, carbonic acid, picric acid, methanesulfonic acid, ethanesulfonic acid, and glutamic acid. Solifenacin succinate may be used in a preferred embodiment of the present invention. According to the present invention crystalline solifenacin succinate polymorphic form I is particularly preferred.

Example 1 of WO 2005/105795 discloses crystalline solifenacin succinate denominated as solifenacin crystalline form I, which is characterized by an X-ray powder diffraction patter having main peaks expressed as 2-theta at about 3.9, 11.2, 14.3 and 18.8 ±0.2 degrees and further characterized by X-ray diffraction peaks expressed as 2-theta at about 7.6, 19.3, 21.1, 23.2 and 25.2 ±0.2 degrees, as also shown in the XRD pattern 1 illustrated in the ipcom publication number IPCOM000147748D.

The dose of solifenacin or a pharmaceutically acceptable salt thereof may be appropriately determined for each patient in accordance with, for example, the route of administration, symptoms of a disease, the age and the sex of a patient to be treated, or the like. When solifenacin succinate is orally administered to an adult, the daily dose is approximately 0.01 mg/kg to 100 mg/kg as the active ingredient and is administered once or divided into two to four doses per day.

Preferably the unit dose is 6 mg.

Crystalline solifenacin succinate polymorphic form I is preferred as the pharmaceutically active ingredient to be used in the tablet formulations and processes of the present invention. Furthermore, crystalline solifenacin succinate polymorphic form I and tamsulosin HCl are particularly preferred as the pharmaceutically active ingredients to be used in the tablet formulations and processes of the present invention.

The API crystalline solifenacin succinate polymorphic form I used as a starting material in the formulations and the processes according to the present invention is preferably characterized by the PXRD peaks shown in the table below:

| 2-theta values of crystalline polymorphic Form I of Solifenacin succinate (± 0.2°) (the most characteristic 2-theta values are in bold) | |
|---|---|
| 3.7 | 21.0 |

(continued)

| 2-theta values of crystalline polymorphic Form I of Solifenacin succinate (± 0.2°) (the most characteristic 2-theta values are in bold) | |
|---|---|
| **7.4** | **21.7** |
| **11.1** | 22.3 |
| **11.8** | 22.6 |
| **13.5** | 23.0 |
| 14.1 | 23.8 |
| **14.8** | 25.0 |
| **15.5** | 25.8 |
| 15.8 | 26.1 |
| 17.6 | 26.5 |
| **18.1** | 27.2 |
| 18.5 | 27.5 |
| 19.2 | 28.5 |
| 20.2 | 29.4 |

The X-ray powder diffraction analysis was performed on Panalytical diffractometer (Empyrean v.3, Malvern Panalytical) with a CuKα source (copper radiation of 1.5418 Å), e.g., with following method settings:

| **Settings** | **Solifenacin succinate** |
|---|---|
| 2θ range | 2 - 40° |
| step size | 0.006° |
| time per step | 15 s |
| scan speed | 0.1 °/s |
| spinning | 8 s |

The API crystalline solifenacin succinate polymorphic form I as used as a starting material in the formulations and processes in accordance with the present invention can also be characterized by the PXRD pattern as depicted in Figure 1, which is essentially the same as the patterns disclosed in WO 2008/013851 and ipcom publication number: IPCOM000147748D referred to above.

In a preferred embodiment of the present invention the solifenacin succinate in the solifenacin layer of the tablet according to the present invention is characterized by PXRD peaks expressed as 2-theta at about 7.4, 11.1, 11.8 and 18.1 ±0.2 degrees obtained from the powdered material of the solifenacin layer.

The X-ray powder diffraction analysis was performed on Panalytical diffractometer (Empyrean v.3, Malvern Panalytical) with CuKα source (copper radiation of 1.5418 Å) with following method settings:

| **Settings** | **Tablets** |
|---|---|
| 2θ range | 2-40° |
| step size | 0.006° |
| time per step scan speed | 150 s<br>0.01 °/s |
| spinning | 0.5 s |

In a more preferred embodiment of the present invention the solifenacin succinate in the solifenacin layer of the tablet formulation of the present invention is characterized by PXRD peaks expressed as 2-theta at about 7.4, 11.1, 11.8,

13.5, 14.8, 15.5, 18.1 and 21.7 ±0.2 degrees.

In the most preferred embodiment, the solifenacin succinate in the solifenacin layer of the tablet formulation in accordance with the present invention is characterized by an PXRD pattern which is essentially the same as the PXRD pattern depicted in Figure 2.

The present invention in particular provides the following:

(1) A tablet for oral administration comprising

(i) a first modified release layer comprising tamsulosin or a pharmaceutically acceptable salt thereof, a matrix forming polymer, and optionally one or more excipients selected from a hydrophilic additive, a diluent, a glidant and a lubricant;
(ii) a second immediate release layer comprising crystalline solifenacin succinate (solifenacin succinate polymorphic form I) and a combination of silicified microcrystalline cellulose (SMCC) and Dicalcium phosphate (DCP) or anhydrous Dicalcium phosphate (DCPA) at a ratio of 2.0-3.8 : 0.7-1.3, preferably at a ratio of 3:1

and optionally one or more excipient selected from a disintegrant, a binder and a lubricant,

wherein the tablet is optionally film coated.

(2) Tablet according to (1) above, which is a bi-layered tablet.
(3) Tablet according to (1) or (2) above, wherein the first modified release layer comprises tamsulosin HCl.
(4) Tablet according any one of (1)-(3) above, wherein the matrix forming polymer is Macrogol 7 000 000.
(5) Tablet according to any one of (1)-(4) above, wherein the first modified release layer comprises microcrystalline cellulose type 200, colloidal anhydrous silica and magnesium stearate.
(6) Tablet according to any one of (1)-(5) above, wherein the second immediate release layer comprises low-substituted hydroxypropyl cellulose and magnesium stearate.
(7) Tablet according to any one of (1)-(6) above, having the following composition

| **Modified release layer** | % of total tablet mass | **Corresponding to % of mass of modified release layer** |
|---|---|---|
| Tamsulosin hydrochloride | 0.114 | 0.16 |
| Macrogol 7 000 000 | 42-67.86 | 60-95 |
| Cellulose, Microcrystalline type 200 | 3.71-25 | 5-35 |
| Silica, Colloidal Anhydrous | 0.071-0.71 | 0.1-1.0 |
| Magnesium stearate | 0.071-2.14 | 0.1-3.0 |
| **Immediate release layer** | | **Corresponding to % of mass of immediate release layer** |
| Crystalline Solifenacin succinate (Crystalline Solifenacin succinate Polymorphic form I) | 1.714 | |
| DCP or DCPA | 5-12 | 15-36 |
| Silicified Microcrystalline Cellulose | 8.57-21.14 | 30-95 |
| Low-Substituted Hydroxypropyl Cellulose | 0.28-1.142 | 1-4 |
| Magnesium stearate | 0.0285-0.857 | 0.1-3.0 |
| Total mass of uncoated tablet: | 100.00 | |
| **Film coating (optional)** | | |
| Opadry® complete Film coating system 03F45072 RED | 3.00 | |
| Total mass of film-coated tablet | 103.00 | |

(8) Tablet according to any one of (1)-(7) above, having the following composition

| **Modified release layer** | % of total tablet mass | Corresponding to % of mass of modified release layer |
|---|---|---|
| Tamsulosin hydrochloride | 0.114 | 0.16 |
| Macrogol 7 000 000 | 57.143 | 80 |
| Cellulose, Microcrystalline type 200 | 13.429 | 18.80 |
| Silica, Colloidal Anhydrous | 0.371 | 0.52 |
| Magnesium stearate | 0.371 | 0.52 |
| **Immediate release layer** | | Corresponding % of mass of immediate release layer |
| Crystalline Solifenacin succinate (Crystalline Solifenacin succinate polymorphic form I) | 1.714 | 6% |
| Dibasic Calcium Phosphate Anhydrous | 6.571 | 23% |
| Silicified Microcrystalline Cellulose | 19.571 | 68.5% |
| Low-Substituted Hydroxypropyl Cellulose | 0.571 | 2% |
| Magnesium stearate | 0.143 | 0.5% |
| Total mass of uncoated tablet: | 100.00 | |
| **Film coating (optional)** | | |
| Opadry® complete Film coating system 03F45072 RED | 3.00 | |
| Total mass of film-coated tablet: | 103.00 | |

(9) Tablet of any one of (1)-(8) above, having the following composition

| | mg/tablet |
|---|---|
| **Modified release layer** | |
| Tamsulosin hydrochloride | 0.4 |
| Macrogol 7 000 000 | 200.0 |
| Cellulose, Microcrystalline type 200 | 47.0 |
| Silica, Colloidal Anhydrous | 1.3 |
| Magnesium stearate | 1.3 |
| **Immediate release layer** | |
| Crystalline Solifenacin succinate (Crystalline Solifenacin succinate polymorphic form I) | 6.0 |
| Dibasic Calcium Phosphate Anhydrous | 23.0 |
| Silicified Microcrystalline Cellulose | 68.5 |
| Low-Substituted Hydroxypropyl Cellulose | 2.0 |
| Magnesium stearate | 0.5 |
| Total mass of uncoated tablet: | 350.0 |
| **Film coating (optional)** | |
| Opadry® complete Film coating system 03F45072 RED | 10.5 |
| Total mass of film-coated tablet: | 360.5 |

(10) Tablet according to any one of (1)-(9) above, wherein the SMCC has a BET of about 6 $m^2/g$ and a mean particle

size of about 125 μm.

(11) Tablet according to any one of (1)-(10) above, wherein the DCP or DCPA has a BET of greater than 35 $m^2/g$, preferably of about 40 $m^2/g$ and mean particle size of 115 -120 μm.

(12) Tablet according to any one of (1)-(11) above, wherein the SMCC has a BET of about 6 $m^2/g$ and a mean particle size of about 125 μm and the DCP or DCPA has a BET of greater than 35 $m^2/g$, preferably about 40 $m^2/g$ and a mean particle size of about 115-120 μm.

(13) Tablet according to any one of (1)-(12) above, wherein at least 94.5% crystalline solifenacin succinate (crystalline solifenacin succinate polymorphic form I) is dissolved after 10 min in accordance with a dissolution test in 0.1 M HCl (pH 1.2), 500 ml, 100 RPM, temperature 37 °C ± 0.5 °C, Apparatus type: 1 (basket, according to European Pharmacopoeia $8^{th}$ edition).

(14) Tablet according to any one of (1)-(13) above wherein at least 95.3% crystalline solifenacin succinate (crystalline solifenacin succinate polymorphic form I) is dissolved after 15 min in accordance with the dissolution test in 0.1 M HCl (pH 1.2), 500 ml, 100 RPM, temperature 37 °C ± 0.5 °C, Apparatus type: 1 (basket, according to European Pharmacopoeia $8^{th}$ edition).

(15) Tablet according to any one of (1)-(14) above, wherein at least 96.9% crystalline solifenacin succinate (crystalline solifenacin succinate polymorphic form I) is dissolved after 30 min in accordance with the dissolution test in 0.1 M HCl (pH 1.2), 500 ml, 100 RPM, temperature 37 °C ± 0.5 °C, Apparatus type: 1 (basket, according to European Pharmacopoeia $8^{th}$ edition).

(16) Tablet according to any one of (1)-(15) above, wherein at least 18.7% tamsulosin is dissolved after 2 h in accordance with the dissolution test in phosphate buffer pH 6.8, 500 ml, 50 RPM, temperature 37 °C ± 0.5 °C, Apparatus type: 2 (paddles with sinkers, according to Japan Pharmacopoeia $15^{th}$ edition).

(17) Tablet according to any one of (1)-(16) above, wherein at least 48.9 tamsulosin is dissolved after 8 h in accordance with the dissolution test in phosphate buffer pH 6.8, 500 ml, 50 RPM, temperature 37 °C ± 0.5 °C, Apparatus type: 2 (paddles with sinkers, according to Japan Pharmacopoeia $15^{th}$ edition).

(18) Tablet according to any one of (1)-(17) above, wherein at least 87.7% of tamsulosin is dissolved after 21 h in accordance with the dissolution test in accordance with the dissolution test in phosphate buffer pH 6.8, 500 ml, 50 RPM, temperature 37 °C ± 0.5 °C, Apparatus type: 2 (paddles with sinkers, according to Japan Pharmacopoeia $15^{th}$ edition).

(19) Process for preparing a tablet according any one of (1)-(18) above, comprising the step of

(I) preparing granulate for the immediate release layer comprising crystalline solifenacin succinate (crystalline solifenacin succinate polymorphic form I) by

(a) mixing crystalline solifenacin succinate (crystalline solifenacin succinate polymorphic form I), SMCC and the binder/disintegrant or disintegrant in a granulator, preferably in a high shear granulator;
(b) spraying the blend obtained in (a) with granulation solvent and granulate;
(c) passing the wet granulate obtained in step (b) through 5 mm sieve and drying the granulate in a fluid bed dryer;
(d) passing the dried granulate through 1 mm mesh;
(e) stepwise adding the granulate external phase - DCP or DCPA - and blending thoroughly and
(f) adding lubricant through 0.5 mm sieve to the blend obtained in step (e); and blending thoroughly;
or

(Ia) preparing granulate for the immediate release layer comprising crystalline solifenacin succinate (crystalline solifenacin succinate polymorphic form I) by

(g) mixing crystalline solifenacin succinate (crystalline solifenacin succinate polymorphic form I) and the water-insoluble excipients DCP or DCPA and SMCC in a granulator, preferably in a high shear granulator;
(h) spraying the blend obtained in (g) with granulation solvent and granulate;
(i) passing the wet granulate obtained in step (h) through 5 mm sieve and drying the granulate in a fluid bed dryer;
(j) passing the dried granulate through 1 mm mesh;
(k) stepwise adding the granulate external phase (binder/disintegrant, disintegrant) and blending thoroughly and
(l) adding the lubricant through 0.5 mm sieve to the blend obtained in step (k); and blending thoroughly.

(20) Process for preparing a tablet according to any one of (1) to (19) above comprising the following steps:

(I) preparing granulate for the immediate release layer comprising crystalline solifenacin succinate (crystalline solifenacin succinate polymorphic form I) by

(a) mixing crystalline solifenacin succinate (crystalline solifenacin succinate polymorphic form I), SMCC and the

binder/disintegrant, disintegrant in a granulator, preferably in a high shear granulator;
(b) spraying the blend obtained in (a) with granulation solvent and granulate;
(c) passing the wet granulate obtained in step (b) through 5 mm sieve and drying the granulate in a fluid bed dryer;
(d) passing the dried granulate through 1 mm mesh;
(e) stepwise adding the granulate external phase - DCP or DCPA - and blending thoroughly and
(f) adding the lubricant through 0.5 mm sieve to the blend obtained in step (e); and blending thoroughly;
or

(Ia) preparing granulate for the immediate release layer comprising crystalline solifenacin succinate (crystalline solifenacin succinate polymorphic form I) by

(g) mixing crystalline solifenacin succinate (crystalline solifenacin succinate polymorphic form I) and the water-insoluble excipients DCP or DCPA and SMCC in a granulator, preferably in a high shear granulator;
(h) spraying the blend obtained in (g) with granulation solvent and granulate;
(i) passing the wet granulate obtained in step (h) through 5 mm sieve and drying the granulate in a fluid bed dryer;
(j) passing the dried granulate through 1 mm mesh;
(k) stepwise adding the granulate external phase - binder/disintegrant or disintegrant and blending thoroughly and
(i) adding lubricant through 0.5 mm sieve to the blend obtained in step (k); and blending thoroughly;

(II) preparing granulate for the modified release layer comprising tamsulosin HCl by

(m) dissolving tamsulosin HCl in the granulation solvent thereby obtaining a granulation liquid;
(n) spraying the solution obtained in step (m) onto the matrix forming polymer Macrogol 7 000 000 using a fluid bed granulator to obtain a granulate;
(o) sieving the obtained (dry) granulate through a 1 mm mesh;
(p) stepwise adding the granulate external phase (diluent and glidant), and blending thoroughly and
(q) adding the lubricant through 0.5 mm sieve to the blend obtained in step (p) and blending thoroughly;

(II) compressing granulates obtained in steps (I) or (Ia) and (II) into bilayer tablets using a bilayer tableting machine and

(III) optionally film coating the tablet cores obtained in step (III) to a weight gain of about 3%.

(21) Process according to (20) above, comprising steps (I), (II), (III) and (IV).
(22) Tablet obtained by a process according to any one of (19) to (21) above.
Thus, the present invention provides the following:

- A tablet as set out above, wherein the modified release layer comprises tamsulosin or a pharmaceutically acceptable salt thereof, in particular tamsulosin HCl, Macrogol 7 000 000, microcrystalline cellulose type 200, colloidal anhydrous silica and magnesium stearate, wherein the tablet is optionally film coated.

- A tablet as set out above, wherein the immediate release layer comprises solifenacin or a pharmaceutically acceptable salt thereof, in particular solifenacin succinate, even more particular crystalline solifenacin succinate polymorphic form I, anhydrous dibasic calcium phosphate, silicified microcrystalline cellulose, low-substituted hydroxypropyl cellulose and magnesium stearate, wherein the tablet is optionally film coated.

- A tablet as set out above, wherein the modified release layer comprises tamsulosin or a pharmaceutically acceptable salt thereof, in particular tamsulosin HCl, Macrogol 7 000 000, microcrystalline cellulose type 200, colloidal anhydrous silica and magnesium stearate and

  wherein the immediate release layer comprises solifenacin or a pharmaceutically acceptable salt thereof, in particular solifenacin succinate, anhydrous dibasic calcium phosphate, silicified microcrystalline cellulose, low-substituted hydroxypropyl cellulose and magnesium stearate, and
  wherein the tablet is optionally film coated.

  - A bilayer tablet according to the present invention in particular comprises a modified release layer comprising 0.4 mg to 1.6 mg of tamsulosin HCl and an immediate release layer comprising 1 to 30 mg of solifenacin succinate. According to the present invention the preferred dosage of tamsulosin HCl is 0.4 mg and of solifenacin succinate

is 6 and 10 mg, whereby 6 mg is more preferred.

- In a preferred embodiment the tablets according the present invention comprise 0.4 mg tamsulosin HCl in the modified release layer and 6 mg solifenacin succinate in the immediate release layer.
- In all embodiments of the invention it is preferred that the second immediate release layer comprises crystalline solifenacin succinate, (preferably polymorphic form I) and a combination of silicified microcrystalline cellulose (SMCC) with a mean particle size of 125μm and a BET of about 6$m^2$/g and dicalcium phosphate (DCP) or anhydrous dicalcium phosphate (DCPA) with a BET of about 40$m^2$/g and a mean particle size of about 115 to about 120 μm at a ratio of 2.0-3.8 : 0.7-1.3, preferably at a ratio of 3:1, wherein the use of DCPA is most preferred.

In the following compositions of the invention are exemplified.

Formulation Examples

According to the invention the formulations of the individual layers can be combined as follows:

A formulation as described in Section 1.1 (modified release layer, in particular according to Section 1.1.1) with any of the formulations according to Section 2.1 - 2.7 (immediate release) and a formulation according to Section 1.2 (modified release layer comprising PEG 8 000, in particular according to Section 1.2.1) with any of the formulations according to Sections 2.1-2.6 (immediate release).

Combinations of formulations as set out in Section 1.1 with formulations as set out in Section 2.1 are preferred, a combination of a formulation based on the example in Section 1.1.1 for the modified release layer with formulation example based on Section 2.1 for the immediate release layer is particularly preferred.

### 1. Composition of the Tamsulosin layer (modified release layer)

| | |
|---|---|
| Tamsulosin HCl | 0.16% (w/w of the layer) |
| Macrogol 7 000 000 | 60 - 95 % (w/w of the layer) |
| PEG 8 000 | 0-35 % (w/w of the layer) |
| MCC type 200 | 0 - 35 % (w/w of the layer) |
| Silica, Colloidal Anhydrous | 0.1 - 1.0 % (w/w of the layer) |
| Magnesium stearate | 0. 1 - 3.0 % (w/w of the layer) |
| Granulation liquid q.s. | |

### 1.1. Embodiment 1 (without PEG 8 000)

In a preferred embodiment the modified release layer does not contain PEG 8 000 and comprises the following:

| | |
|---|---|
| Tamsulosin HCl | 0.16% (w/w of the layer) |
| Macrogol 7 000 000 | 60 - 95 % (w/w of the layer) |
| MCC type 200 | 5 - 35 % (w/w of the layer) |
| Silica, Colloidal Anhydrous | 0.1 - 1.0 % (w/w of the layer) |
| Magnesium stearate | 0. 1 - 3.0 % (w/w of the layer) |
| Granulation liquid q.s. | |

### 1.1.1 Formulation example 1.1.1 (preferred)

| | mg per unit | % (w/w) layer |
|---|---|---|
| Tamsulosin hydrochloride | 0.4 | 0.16 |
| Macrogol 7 000 000 | 200.0 | 80.00 |
| Cellulose, Microcrystalline type 200 | 47.0 | 18.80 |

(continued)

| | mg per unit | % (w/w) layer |
|---|---|---|
| Silica, Colloidal Anhydrous | 1.3 | 0.52 |
| Magnesium stearate | 1.3 | 0.52 |
| Granulation solvent Ethanol 96% (v/v): water (70:30) | qs | - |

The above formulation may generally be prepared as follows:
The API is dissolved in the granulation solvent using a mixer. Macrogol 7 000 000 is placed into a fluid bed granulator (such as Glatt WS Combo) and the fluid bed is heated to about 35 °C. The granulation liquid is then sprayed onto the Macrogol keeping the bed temperature above 30 °C. The obtained granulate is sieved through a 1mm mesh sieve. Then the granulate external phase - cellulose, silica is added and blended for 20 min using a tumble blender. Then the lubricant is added through 0.5 mm sieve to the granulate and mixed for 5 min.

**1.2.1 Further Embodiment 2 comprising PEG 8000**

| | |
|---|---|
| Tamsulosin hydrochloride | 0.165 % (w/w of the layer) |
| Macrogol 7 000 000 | 60 - 95 % (w/w of the layer) |
| PEG 8 000 | 5 - 35 % (w/w of the layer) |
| Silica, Colloidal Anhydrous | 0.1 - 1.0% (w/w of the layer) |
| Magnesium stearate | 0.1 - 3.0% (w/w of the layer) |
| Granulation solvent | q.s. |

**1.2.1 Formulation example 1.2.1 (particular embodiment of 1.2)**

| | mg per unit | % layer |
|---|---|---|
| Tamsulosin hydrochloride | 0.4 | 0.165 |
| Macrogol 7 000 000 | 200.0 | 82.508 |
| PEG 8000 | 40.0 | 16.502 |
| Silica, Colloidal Anhydrous | 10 | 0.413 |
| Magnesium stearate | 1.0 | 0.413 |
| Granulation solvent Ethanol 96% (v/v): water (70:30) | qs | - |

The above formulation may generally be prepared as follows:
API and 30% of PEG 8000 are dissolved in the granulation solvent using a mixer. Macrogol 7 000 000 and the rest of PEG 8000 are placed into a fluid bed granulator (such as Glatt WS Combo). The fluid bed is heated to about 35 °C. The granulation liquid is then sprayed on the Macrogol and PEG 8000 keeping the bed temperature above 30 °C. The obtained granulate is sieved through a 1 mm mesh sieve. The granulate external phase anhydrous colloidal silica is added and blended for 20 min using tumble blender. Then the lubricant is added through a 0.5 mm sieve to the granulate and mixed for 5 min.

**2. Composition of the Solifenacin layer (immediate release layer)**

| | |
|---|---|
| Solifenacin succinate<br>(crystalline solifenacin succinate polymorphic form I) | 6.0 % (w/w of the layer) |

(continued)

| | |
|---|---|
| Water insoluble diluent selected from DCP and DCPA | 15-36% (w/w of the layer), |
| Silicified Microcrystalline Cellulose (SMCC) | 30 - 95 % (w/w of the layer), 55-76% |
| Disintegrant | |
| selected from Low-Substituted Hydroxypropyl Cellulose(L-HPC) and Croscarmellose sodium | 1-4 % (w/w of the layer) |
| Lubricant | 0.1-3.0 % (w/w of the layer) |
| Magnesium stearate | |
| Granulation solvent | q.s |
| ethanol 96% (v/v) or ethanol 96%: | water (70:30) |

**2.1 Formulation example 2.1 (preferred embodiment)**

| | mg per unit | % (w/w) layer |
|---|---|---|
| Solifenacin succinate (Crystalline Solifenacin succinate polymorphic form I) | 6.0 | 6.0 |
| DCP or DCPA | 23.0 | 23.0 |
| Silicified Microcrystalline Cellulose | 68.5 | 68.5 |
| Low-Substituted Hydroxypropyl Cellulose | 2.0 | 2.0 |
| Magnesium stearate | 0.5 | 0.5 |
| Granulation solvent Ethanol 96% (v/v): water (70:30) | q.s. | - |

The formulation may generally be prepared as follows:
All excipients are passed through a 1 mm mesh. API, DCP or DCPA and SMCC are placed into a high shear granulator (such as Glatt VG Pro) and mixed for 10 min. The mix is sprayed with the granulation solvent for 5 min and granulate for 1 min. The wet granulate is then sieved through a 5 mm sieve. The granulate is dried to a LOD below 5% using fluid bed dryer (such as Glatt WS Combo) and passed through a 1 mm mesh. Then the granulate external phase -the disintegrant L-HPC is added and mixed for 20 min using a tumble blender. Magnesium stearate is then added through a 0.5 mm sieve and blended for 5 min.

Further particular embodiments of the formulation for the immediate release layer are disclosed in 2.2 to 2.6, below, which may also be prepared as described for example 2.1, above.

**2.2 Formulation Example 2.2**

| | mg per unit | % (w/w) layer |
|---|---|---|
| Solifenacin succinate (Crystalline Solifenacin succinate polymorphic form I) | 6.0 | 6.00 |
| DCP or DCPA | 40.0 | 40.00 |
| Silicified Microcrystalline Cellulose | 51.0 | 51.00 |
| Croscarmellose sodium | 2.0 | 2.00 |
| Magnesium stearate | 1.0 | 1.00 |
| Granulation solvent Ethanol 96% (v/v): water (70:30) | q.s | - |

**2.3 Formulation example 2.3**

| | mg per unit | % (w/w) layer |
|---|---|---|
| Solifenacin succinate (Crystalline Solifenacin succinate polymorphic form I) | 6.0 | 6.0 |
| DCP or DCPA | 10.0 | 10.0 |
| Silicified Microcrystalline Cellulose | 81.0 | 81.0 |
| Low-Substituted Hydroxypropyl Cellulose | 2.0 | 2.0 |
| Magnesium stearate | 1.0 | 1.0 |
| Granulation solvent Ethanol 96% (v/v): water (70:30) | q.s. | - |

**2.4 Formulation Example 2.4**

| | mg per unit | % (w/w) layer |
|---|---|---|
| Solifenacin succinate (Crystalline Solifenacin succinate polymorphic form I) | 6.0 | 6.0 |
| DCP or DCPA | 15.0 | 15.0 |
| Sillicified Microcrystalline Cellulose | 76.0 | 76.0 |
| Croscarmellose sodium | 2.0 | 2.0 |
| Magnesium stearate | 1.0 | 1.0 |
| Granulation solvent Ethanol 96% (v/v): water (70:30) | q.s. | - |

**2.5 Formulation example 2.5**

| | mg per unit | % (w/w) layer |
|---|---|---|
| Solifenacin succinate (Crystalline Solifenacin succinate polymorphic form I) | 6.0 | 6.0 |
| DCP or DCPA | 30.0 | 30.0 |
| Silicified Microcrystalline Cellulose | 61.0 | 61.0 |
| Low-Substituted Hydroxypropyl Cellulose | 2.0 | 2.0 |
| Magnesium stearate | 1.0 | 1.0 |
| Granulation solvent Ethanol 96% (v/v): water (70:30) | q.s. | - |

**2.6 Formulation example 2.6**

| | mg per unit | % (w/w) layer |
|---|---|---|
| Solifenacin succinate (Crystalline Solifenacin succinate polymorphic form I) | 6.0 | 6.0 |
| Dibasic Calcium Phosphate, anhydrous | 20.0 | 20.0 |
| Silicified Microcrystalline Cellulose | 71.0 | 71.0 |
| Low-Substituted Hydroxypropyl Cellulose (L-HPC) | 2.0 | 2.0 |
| Magnesium stearate | 1.0 | 1.0 |
| Granulation solvent Ethanol 96% (v/v): water (70:30) | q.s. | - |

This formulation as well as also formulations examples 2.1 to 2.5, above may generally also be prepared as follows:
All excipients are passed through a 1 mm mesh. API, SMCC (such as Prosolv 90 HD) and L-HPC are placed into a high shear granulator (such as Glatt VG Pro) and mixed for 10 min with 120 rpm. The mix is sprayed with granulation solvent for 5 min and granulated for 3 min. The wet granulate is then sieved through a 5 mm sieve and dried to a LCD below 5% using a fluid bed dryer (such as Glatt WS Combo). The granulate is passed through a 1 mm mesh the granulate external phase - DCPA (such as Fujicalin) is added and mixed for 20 min using tumble blender. Magnesium stearate (such as Ligamed MF 2V) is then added through 0.5 mm sieve and blended for 5 min.

**Compression to Tamsulosin / Solifenacin tablets**

Compress both blends into bilayer tablets using bilayer tableting machine (such as Fette 3090 or Kilian KTP). Check tensile strength, friability and uniformity of mass (20 tablets), desired values are 1.0-5.0 MPa; < 1% ±5% (min 18 measurements) and ±10.0 (maximum 2 measurements) for the whole tablet. Optionally coat the tablet cores to a weight gain of about 3% with a tablet coater such as Glatt GC Smart equipped with full perforated coating drum.

**Formulation example 3 (most preferred formulation)**

The following formulation was used for the dissolution tests, accelerated conditions, bioequivalence tests and comparison with prior art:

| Component | | Quantity | | Function | Reference to Quality Standard |
|---|---|---|---|---|---|
| | | mg/tab | %/tab | | |
| Tamsulosin layer | Tamsulosin hydrochloride | 0.4 | 0.114 | Active substance | Ph. Eur. |
| | Macrogol 7 000 000 | 200.0 | 57.143 | Diluent / Matrix former | Ph. Eur. + In-house Material Specification |
| | Ethanol | qs | - | Granulation liquid | Ph. Eur. |
| | Water, Purified | qs | - | Granulation liquid | Ph. Eur. |
| | Cellulose, Microcrystalline type 200 | 47.0 | 13.429 | Diluent | Ph. Eur. |
| | Silica, Colloidal Anhydrous | 1.3 | 0.371 | Glidant | Ph. Eur. |
| | Magnesium stearate | 1.3 | 0.371 | Lubricant | Ph. Eur. |
| Solifenacin layer | Solifenacin succinate (crystalline polymorphic form I) | 6.0 | 1.714 | Active substance | Ph. Eur. |
| | Dibasic Calcium Phosphate Anhydrous (Fujucalin) | 23.0 | 6.571 | Diluent | Ph. Eur. |
| | Silicified Microcrystalline Cellulose (Prosolv 90 HD) | 68.5 | 19.571 | Diluent | In-house Material Specification |
| | Low-Substituted Hydroxypropyl Cellulose | 2.0 | 0.571 | Disintegrant / Binder | Ph. Eur. |
| | Ethanol 96% (v/v): water (70:30) | qs | - | Granulation liquid | Ph. Eur. |
| | Magnesium stearate | 0.5 | 0.143 | Lubricant | Ph. Eur. |
| Total mass of uncoated tablet: | | 350.0 | 100.00 | - | - |

| | | | | | |
|---|---|---|---|---|---|
| Coating | Film coating system<br><br>Opadry® complete coating system 03F45072 RED | 10.5 | 3.00 | Film coating agent | In-house Material Specification |
| | Water, Purified | qs | qs | Coating liquid | Ph. Eur. |
| Total mass of film-coated tablet: | | 360.5 | 103.00 | - | - |

Thus, the compositions of the invention will preferably comprise the listed compounds in the ratios specified below.

| **Modified release layer** | % of total tablet mass | **Corresponding to % of mass of modified release layer** |
|---|---|---|
| Tamsulosin hydrochloride | 0.114 | 0.16 |
| Macrogol 7 000 000 | 42-67.86 | 60-95 |
| Cellulose, Microcrystalline type 200 | 3.71-25 | 5-35 |
| Silica, Colloidal Anhydrous | 0.071-0.71 | 0.1-1.0 |
| Magnesium stearate | 0.071-2.14 | 0.1-3.0 |
| **Immediate release layer** | | **Corresponding to % of mass of immediate release layer** |
| Solifenacin succinate (Crystalline Solifenacin succinate polymorphic form I) | 1.714 | |
| DCP or DCPA | 5-12 | 15-36 |
| Silicified Microcrystalline Cellulose | 8.57-21.14 | 30-95 |
| Low-Substituted Hydroxypropyl Cellulose | 0.28-1.142 | 1-4 |
| Magnesium stearate | 0.0285-0.857 | 0.1-3.0 |
| Total mass of uncoated tablet: | 100.00 | |
| **Film coating (optional)** | | |
| e.g. Opadry® complete Film coating system 03F45072 RED | 3.00 | |
| Total mass of film-coated tablet: | 103.00 | |

The tablet according the present invention, in particular, may have the following composition:

| **Modified release layer** | % of total tablet mass | Corresponding to % of mass of modified release layer |
|---|---|---|
| Tamsulosin hydrochloride | 0.114 | 0.16 |
| Macrogol 7 000 000 | 57.143 | 80 |
| Cellulose, Microcrystalline type 200 | 13.429 | 18.80 |
| Silica, Colloidal Anhydrous | 0.371 | 0.52 |
| Magnesium stearate | 0.371 | 0.52 |
| **Immediate release layer** | | Corresponding % of mass of immediate release layer |
| Solifenacin succinate (Crystalline Solifenacine succinate polymorphic form I) | 1.714 | 6 |
| DPC or DCPA | 6.571 | 23 |
| Silicified Microcrystalline Cellulose | 19.571 | 68.5 |
| Low-Substituted Hydroxypropyl Cellulose | 0.571 | 2 |
| Magnesium stearate | 0.143 | 0.5 |
| Total mass of uncoated tablet: | 100.00 | |

(continued)

| | | |
|---|---|---|
| **Film coating (optional)** | | |
| e.g. Opadry® complete Film coating system 03F45072 RED | 3.00 | |
| Total mass of film-coated tablet: | 103.00 | |

A particular preferred tablet in accordance with the present invention is disclosed below:

| | mg/tablet |
|---|---|
| **Modified release layer** | |
| Tamsulosin hydrochloride | 0.4 |
| Macrogol 7 000 000 | 200.0 |
| Cellulose, Microcrystalline type 200 | 47.0 |
| Silica, Colloidal Anhydrous | 1.3 |
| Magnesium stearate | 1.3 |
| **Immediate release layer** | |
| Solifenacin succinate (Crystalline Solifenacin succinate polymorphic form I) | 6.0 |
| Dibasic Calcium Phosphate Anhydrous (DCPA) | 23.0 |
| Silicified Microcrystalline Cellulose | 68.5 |
| Low-Substituted Hydroxypropyl Cellulose | 2.0 |
| Magnesium stearate | 0.5 |
| Total mass of uncoated tablet: | 350.0 |
| **Film coating (optional)** | |
| e.g. Opadry® complete Film coating system 03F45072 RED | 10.5 |
| Total mass of film-coated tablet: | 360.5 |

Furthermore, according to the present invention provides tablet formulation as described above wherein

- at least 94.5% solifenacin is dissolved after 10 min in accordance with a dissolution test in 0.1 M HCl (pH 1.2), 500 ml, 100 RPM, temperature 37°C +- 0.5°C, Apparatus type: 1 (basket, according to European Pharmacopoeia 8$^{th}$ edition).

- at least 95.3% solifenacin is dissolved after 15 min in accordance with the dissolution test in 0.1 M HCl (pH 1.2), 500 ml, 100 RPM, temperature 37°C +- 0.5°C, Apparatus type: 1 (basket, according to European Pharmacopoeia 8$^{th}$ edition).

- at least 96.9% solifenacin is dissolved after 30 min in accordance with the dissolution test in 0.1 M HCl (pH 1.2), 500 ml, 100 RPM, temperature 37°C +- 0.5°C, Apparatus type: 1 (basket, according to European Pharmacopoeia 8$^{th}$ edition).

As noted above, also the dissolution profile of tamsulosin from the tablet according to the invention is not compromised when compared to the dissolution profile on of the approved mono preparation.

Thus, the present invention provides tablets wherein at least 18.7% tamsulosin is dissolved after 2 h in accordance with the dissolution test in phosphate buffer pH 6.8, 500 ml, 50 RPM, temperature 37°C +- 0.5°C, Apparatus type: 2 (paddles with sinkers, according to Japan Pharmacopoeia 15$^{th}$ edition);

- wherein at least 48.9 tamsulosin is dissolved after 8 h in accordance with the dissolution test in phosphate buffer pH 6.8, 500 ml, 50 RPM, temperature 37 °C +-0.5 °C, Apparatus type: 2 (paddles with sinkers, according to Japan Pharmacopoeia 15$^{th}$ edition);

- wherein at least 87.7% of tamsulosin is dissolved after 21 h in accordance with the dissolution test in accordance with the dissolution test in phosphate buffer pH 6.8, 500 ml, 50 RPM, temperature 37 °C +- 0.5 °C, Apparatus type: 2

(paddles with sinkers, according to Japan Pharmacopoeia 15th edition).

**Process for producing the tablets**

As stated above, the present invention also provides a process for the production of the tablets the present invention as disclosed in more detail below.

Solifenacin succinate/Tamsulosin hydrochloride, modified-release tablets according to the present invention are manufactured with fluid bed granulation (modified release layer), high shear granulation (immediate release layer), fluid bed drying, compression and optionally coating with an aesthetic coating such as film-coating.

The following types of manufacturing equipment may be used for producing the Solifenacin succinate/Tamsulosin hydrochloride, modified-release tablets according to the present invention

| Process step | Equipment |
|---|---|
| Fluid Bed Granulation | Fluid Bed Granulator |
| Particle size reduction/separation | Screening Mills / Impact Mills / Hand Sieving / Vibratory/Shaker Separator |
| Blending | Diffusion Mixers (Tumble) |
| Tabletting | Multi-layer tablet press |
| Coating | Pan Coating |
| Packaging | Heat Sealer |

The present invention provides a process for preparing a tablet according to the present invention comprising the step of

(1) preparing granulate for the immediate release layer comprising solifenacin succinate by

(a) mixing solifenacin succinate, SMCC and the binder/disintegrant or disintegrant in a granulator, preferably a high shear granulator;
(b) spraying the blend obtained in (a) with granulation solvent and granulate;
(c) passing the wet granulate obtained in step (b) through 5 mm sieve and drying the granulate in a fluid bed dryer;
(d) passing the dried granulate through 1 mm mesh;
(e) stepwise adding the granulate external phase - DCP or DCPA - and blending thoroughly and
(f) adding the lubricant (such as Mg stearate) through 0.5 mm sieve to the blend obtained in step (e); and blending thoroughly;
or

(1a) preparing granulate for the immediate release layer comprising solifenacin succinate by

(g) mixing solifenacin succinate and the water-insoluble excipients DCP or DCPA and SMCC in a granulator, preferably a high shear granulator;
(h) spraying the blend obtained in (g) with granulation solvent and granulate;
(i) passing the wet granulate obtained in step (h) through 5 mm sieve and drying the granulate in a fluid bed dryer;
(j) passing the dried granulate through 1 mm mesh;
(k) stepwise adding the granulate external phase (binder/disintegrant or disintegrant) and blending thoroughly and
(l) adding the lubricant (such as Mg stearate) through 0.5 mm sieve to the blend obtained in step (k); and blending thoroughly;

A process comprising step (1) is preferred.

Moreover, the present invention provides a process for preparing a tablet according to the present invention comprising the following steps:

(1) preparing granulate for the immediate release layer comprising solifenacin succinate by

(a) mixing solifenacin succinate, SMCC and the binder/disintegrant or disintegrant in a granulator, preferably a

high shear granulator;
(b) spraying the blend obtained in (a) with granulation solvent and granulate;
(c) passing the wet granulate obtained in step (b) through 5 mm sieve and drying the granulate in a fluid bed dryer;
(d) passing the dried granulate through 1 mm mesh;
(e) stepwise adding the granulate external phase - DCP or DCPA - and blending thoroughly and
(f) adding the lubricant (such as Mg stearate) through 0.5 mm sieve to the blend obtained in step (e); and blending thoroughly;
or

(1a) preparing granulate for the immediate release layer comprising solifenacin succinate by

(g) mixing solifenacin succinate and the water-insoluble excipients DCP or DCPA and SMCC in a granulator, preferably a high shear granulator;
(h) spraying the blend obtained in (g) with granulation solvent and granulate;
(i) passing the wet granulate obtained in step (h) through 5 mm sieve and drying the granulate in a fluid bed dryer;
(j) passing the dried granulate through 1 mm mesh;
(k) stepwise adding the granulate external phase -binder/disintegrant or disintegrant and blending thoroughly and
(l) adding the lubricant (such as Mg stearate) through 0.5 mm sieve to the blend obtained in step (k); and blending thoroughly;

(2) preparing granulate for the modified release layer comprising tamsulosin HCl by

(m) dissolving tamsulosin HCl in the granulation solvent thereby obtaining a granulation liquid;
(n) spraying the solution obtained in step (m) onto the matrix forming polymer Macrogol 7 000 000 using a fluid bed granulator to obtain a granulate;
(o) sieving the obtained (dry) granulate through a 1 mm mesh;
(p) stepwise adding the granulate external phase (diluent and glidant, such as MCC type 200 and anhydrous colloidal silica) and blending thoroughly and
(q) adding the lubricant (such as Mg stearate) through 0.5 mm sieve to the blend obtained in step (p) and blending thoroughly;

(3) compressing granulates obtained in steps (1) or (1a) and (2) into bilayer tablets using a bilayer tableting machine and

(4) optionally film coating the tablet cores obtained in step (3) to a weight gain of about 3%.

DCP or DCPA may be added to the intra (1a) or extra-granular phase (1) of the immediate release layer. The stepwise addition to the extra-granular phase as disclosed in step 1(e) is preferred, in particular for large scale preparation using a high shear granulator. An increased homogeneity of the admixture and a better flow is observed. However, variants prepared with the brittle water insoluble excipient (DCP or DCPA) in the intragranular phase work also very well in terms of technical process requirements as well as in terms of stability such as (impurities, polymorph stability).
In the alternative embodiment, as described in Example 1.2, the modified release layer comprises PEG 8 000, in this case a portion of PEG 8 000 is dissolved in the granulation solvent in step 2(m) and the remaining amount of PEG 8 000 is added to Macrogol 7 000 000 in step 2(n), the mixture of which is then sprayed with the granulation liquid in step 2(n).
Thus, according to the present invention, a process comprising steps (1), (2), (3) and (4) as disclosed above is preferred.
The fluid bed granulation is preferably carried out under the following conditions:

| Parameter | Requirements |
|---|---|
| Inlet air temperature | 25 - 65°C |
| Air flow | 300 - 1000m$^3$/h or equivalent to 0.5 - 1.8 m/s of air velocity |
| Fluid bed temperature (during spraying) | 13 - 25°C |

The tablets thus obtained may be packaged into blisters consisting of Polyamide - Aluminium - PVC (laminate) and Aluminium foil using a heat sealer.
The particularly preferred embodiment as disclosed in formulation example 3 was subjected to comparative

stability and dissolution tests with Vesomni®, a formulation in accordance with Astellas' patent application EP 2 394 648.

Stability and dissolution

Stability studies with tablets of the invention according to formulation example 3 packed in PA-aluminium-PVC (laminate) and aluminium foil blister and carton box have been carried out at accelerated conditions (25°C/60%RH, 30°C/65%RH, 30°C/75%RH and 40°C/75% RH), the dissolution profiles of the active substances remained substantially unchanged and no changes were observed in the aged formulations.

Stress test

The tablets in accordance with formulation example 3 were stored for 6 and 12 months at 25°C/60%RH and for 6 months at 40°C/75%RH, respectively.

For the analysis first the coating of the tablets was scraped off using a scalpel, then the solifenacin layer of the resulting uncoated bilayer tablet was also scraped off with the use of a scalpel. The powdered fine material obtained from the solifenacin layer was subjected directly to XRPD. The X-ray powder diffraction analysis was performed on Panalytical diffractometer (Empyrean v.3, Malvern Panalytical) with CuKα source (copper radiation of 1.5418 Å) with following methods settings:

| **Settings** | **Tablets according to Formulation Example 3** |
|---|---|
| 2θ range | 2 - 40° |
| step size | 0.006° |
| time per step scan speed | 150 s<br>0.01 °/s |
| spinning | 0.5 s |

| **SOT b.12118718**<br>(Tablets according to Formulation Example 3)<br>time point: 0 | 7.4 | 11.1 | 11.8 | 13.5 | 14.8 | 15.5 | 18.1 | 21.7 |
|---|---|---|---|---|---|---|---|---|
| **SOT b.12118718**<br>6M / 25°C/60%RH | 7.4 | 11.1 | 11.8 | 13.5 | 14.8 | 15.5 | 18.1 | 21.7 |
| **SOT b.12118718**<br>12M / 25°C/60%RH | 7.4 | 11.1 | 11.8 | 13.5 | 14.8 | 15.5 | 18.1 | 21.7 |
| **SOT b.12118718**<br>6M / 40°C/75%RH | 7.4 | 11.1 | 11.8 | 13.5 | 14.8 | 15.45 | 18.1 | 21.7 |

XRD patterns obtained from the solifenacin layer of tablets according to formulation example 3 at time point 0 and after storage in conditions 6M/25°C/60%RH,12M/ 25°C/60%RH and 6M/40°C/75%RH are characterized by the PXRD peaks shown in the table above. The XRD patterns obtained under the afore-mentioned conditions are depicted in Figures 2, 3, 4 and 5 respectively.

It is understood that the peaks considered characteristic for solifenacin succinate form I in the XRD patterns obtained from the solifenacin layers of the tablets of Formulation Example 3 are less intense than in the XRD pattern obtained from the pure API solifenacin succinate form I for various reasons such as:

- the presence of excipients; a diminished intensity or no intensity of some peaks is due to the preferred orientation of solifenacin succinate crystals, and/or the presence of a heavily absorbing element (such as Ca in $CaPO_4$) in the tablet mass (Fawcett, T.G., Gates-Rector, S., Gindhart, A.M., Rost, M., Kabekkodu, S.N., Blanton, J.R., Blanton, T.N. (2019) A practical guide to pharmaceutical analyses using X-ray powder diffraction. Powder Diffr., 34 (2), 164-183.);
- the actual concentration of the API solifenacin succinate form I in the tablet is low (6 mg in 100 mg of the solifenacin layer); and
- as the active substance is wet granulated with excipients, the crystals of the active substance solifenacin succinate are present in close association with excipients, which in turn changes the dimensions of the solifenacin succinate

crystals.

However, as demonstrated by the last-shown table and Figures 2 to 5, the XDR patterns obtained from the solifenacin layer of the tablets at time point 0 and after storage for 6 and 12 months under conditions exhibit the XRD peaks considered characteristic for solifenacin succinate form I, indicating that solifenacin succinate form I remains unchanged in the formulation in accordance with the present invention.

Comparative experiments with prior art formulation Vesomni

Comparative stability studies at accelerated conditions for the SOL/TAM tablets of Example 3, for dose 6 mg/0.4 mg and the prior art formulation according to Astellas' patent application EP 2 394 648 (Vesomni®) have carried out and revealed equivalent stability of both formulations.

The compositions were analyzed after 3 and 6 months of storage at 40 °C, 75% RH in blisters PA-ALU-PVC (laminate)/ALU. The level of impurities was determined by HPLC method.

The results are presented in Table 1, below:

Table 1

| Impurities (limit) | Tablets 6 mg/0.4 mg of Example 3 | | | Vesomni® 17C27/36 | | |
|---|---|---|---|---|---|---|
| | start [%] | 3 months [%] | 6 months [%] | start [%] | 3 months [%] | 6 months [%] |
| Max. single unspecified impurity of TAM (≤ 1.0 %) | ND | 0.29 % | 0.31 % | ND | 0.25 % | 0.38 % |
| Impurity I* of SOL (≤ 0.8 %) | ND | < 0.1 % | < 0.1 % | ND | < 0.1 % | < 0.1 % |
| Max. single unspecified impurity of SOL (≤ 0.3 %)) | ND | 0.22 % | ND | ND | ND | ND |
| Total impurities (≤ 3.0 %) | < 0.1 % | 0.78 % | 0.81 % | < 0.1 % | 0.31 % | 0.80 % |

The requirements for related substances were established according to guidelines CPCP/ICH/367/96 (ICH Q6A), and CPMP/ICH/2738/99 (ICH Q3B (R2)). The in-house chromatographic method for related substances is selective for degradation products.

*Impurity I of Solifenacin (3R)-3-[[(1S)-1-phenyl-3,4-dihydroisoquinoline-2(1H)-carbonyl]oxy]-1-azabicyclo[2.2.2]octane 1-oxide(solifenacin N-oxide)

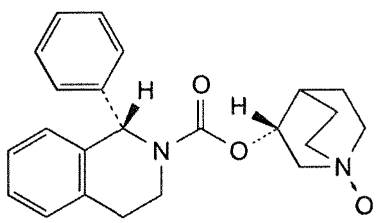

Comparison of the dissolution profiles

Dissolution tests

The pharmaceutical compositions according to the present invention prepared in Formulation Example 3 and the marketed prior art formulation Vesomni® were subjected to dissolution tests carried out in accordance with the test methods described in Table 2, below:

Table 2

| Parameter | Dissolution testing conditions for Tamsulosin Hydrochloride | Dissolution testing conditions for Solifenacin Succinate |
|---|---|---|
| Medium: | Phosphate buffer pH 6.8 | 0.1 M HCl (pH 1.2) |
| Volume [ml]: | 500 | 500 |
| Apparatus type: | Apparatus type: 2 (paddles with sinkers, according to Japan Pharmacopoeia 15th edition) | Apparatus type: 1 (baskets, according to European Pharmacopoeia 8th edition) |
| Temperature | 37°C +- 0.5°C | 37°C +- 0.5°C |
| RPM: | 50 | 100 |
| Time points [min]: | 30, 60, 120, 180, 240, 300, 360, 480, 600, 720, 900, 1080, 1260, 1440 | 10, 15, 20, 30, infinity test |

Dissolution testing for tablets SOL/TAM prepared according Example 3, reference: Solifenacin Succinate / Tamsulosin Hydrochloride tablets 6 mg/0.4 mg (Vesomni®) (Table 3)
- dissolution of Tamsulosin Hydrochloride

Table 3

| Dissolution Data - phosphate buffer pH 6.8 | | | | | |
|---|---|---|---|---|---|
| SOL/TAM tablets, 6 mg/0.4 mg, Example 3 | | | SOL/TAM reference tablets, 6 mg/0.4 mg (Vesomni®) | | |
| Time point [min] | Mean [%] | RSD [%] | Time point [min] | Mean [%] | RSD [%] |
| 0 | 0 | - | 0 | o | - |
| 30 | 7.8 | 4.7 | 30 | 6.7 | 5.8 |
| 60 | 12.8 | 5.8 | 60 | 11.0 | 4.2 |
| 120 | 19.9 | 4.2 | 120 | 18.1 | 3.3 |
| 180 | 26.2 | 4.7 | 180 | 24.2 | 3.4 |
| 240 | 31.7 | 3.6 | 240 | 29.8 | 2.6 |
| 300 | 37.2 | 3.6 | 300 | 35.3 | 2.4 |
| 360 | 42.4 | 3.4 | 360 | 40.8 | 2.8 |
| 480 | 51.9 | 3.1 | 480 | 50.5 | 2.2 |
| 600 | 60.3 | 3.0 | 600 | 59.3 | 1.9 |
| 720 | 67.7 | 2.7 | 720 | 67.0 | 1.9 |
| 900 | 77.0 | 2.3 | 900 | 76.4 | 1.7 |
| 1080 | 84.2 | 2.0 | 1080 | 83.7 | 1.5 |
| 1260 | 89.3 | 1.7 | 1260 | 89.0 | 1.4 |
| 1440 | 93.3 | 1.5 | 1440 | 92.8 | 1.3 |

The Tamsulosin Hydrochloride dissolution profiles from the tested tablet according to the present invention and reference drug product (Vesomni®) were assessed in a buffer at pH 6.8 on the basis of the calculated similarity factor f2. The obtained value of f2 factor, equals 88.0, confirms that the dissolution profiles are similar.

Dissolution testing for tablets SOL/TAM prepared according Example 3, reference: Solifenacin Succinate / Tamsulosin Hydrochloride tablets 6 mg/0.4 mg (Vesomni®) (Table 4)
- dissolution of Solifenacin Succinate

Table 4

| Dissolution Data - 0.1 M HCl pH 1.2 | | | | | |
|---|---|---|---|---|---|
| SOL/TAM tablets, 6 mg/0.4 mg, Example 3 | | | SOL/TAM reference tablets, 6 mg/0.4 mg (Vesomni®) | | |
| Time point [min] | Mean [%] | RSD [%] | Time point [min] | Mean [%] | RSD [%] |
| 0 | 0 | - | 0 | 0 | - |
| 10 | 96.1 | 1.8 | 10 | 82.0 | 6.1 |
| 15 | 97.0 | 1.8 | 15 | 90.0 | 2.0 |
| 20 | 97.7 | 1.8 | 20 | 91.4 | 1.3 |
| 30 | 98.5 | 1.8 | 30 | 92.7 | 1.0 |
| infinity test | 99.6 | 1.7 | infinity test | 93.8 | 1.1 |

The amount of solifenacin succinate dissolved in 0.1 M HCl from the tablet according to the present invention and the reference drug product (Vesomni®) was above 85 % within 15 minutes, thus the dissolution profiles of API from both products are assumed similar without further mathematical evaluation according to "Guideline on the Investigation of Bioequivalence" (CPMP/EWP/QWP/1401/98 Rev. 1/Corr**).

As can be derived from Table 4, above and Table 5, below, the maximum of the dissolution of solifenacin and also the dissolution rate after 15 minutes was improved when compared to the prior art formulation Vesomni® and not delayed by the presence of a modified release layer.

Table 5

| | **REQUIREMENTS** | **SOT 12118718** | **SOT 12118719** | **SOT 12118720** | Vesomni® 18G27/35 |
|---|---|---|---|---|---|
| Dissolution of solifenacin | After 15 minutes:<br>for each tablet ≥ 85% ($S_1$= Q + 5; Q = 80%)<br><br>The additional test is acceptable | After 15 minutes<br>Average value 97 %<br>min. value 95 %<br>max. value 101 % | After 15 minutes<br>Average value 95 %<br>min. value 92 %<br>max. value 97% | After 15 minutes<br>Average value 93 %<br>min. value 91 %<br>max. value 95 % | After 15 minutes<br>Average value 90 %<br>min. value 87%<br>max. value 92 % |

As shown above, the tablet according to the present invention releases the API tamsulosin HCl in the identical manner as the commercially available product, whereas the API solifenacin succinate is released faster from the formulations according to the present invention. Moreover, a dissolution of 100% is achieved for solifenacin succinate.

Additionally, stability testing under accelerated conditions shows that the pharmaceutical composition of the tablet in accordance with the present invention complies with the limits specified for the chemical purity of medical drug product. The increase in impurities levels, which are degradation products of tamsulosin hydrochloride and solifenacin succinate, is negligible and the product meets the requirements for pharmaceutical product.

Bioequivalence studies

Bioequivalence studies were performed in accordance with the current revision of the Declaration of Helsinki, International Council for Harmonisation (ICH), Good Clinical Practice (GCP) guidelines, the EU Clinical Trials Directive 2001/20/EC and applicable regulatory guidelines. The pharmacokinetic studies were performed for Solifenacin Succinate/Tamsulosin Hydrochloride, modified-release tablets, 6mg/0.4mg prepared according to Formulation Example 3 above. The aim of the studies was to characterize the rate and extend of solifenacin and tamsulosin absorption after oral administration and to assess the bioequivalence of the formulation according to Formulation Example 3 of the present invention (test product) and the marketed prior art formulation Vesomni® 6mg/0.4mg modified-release tablets (reference product). Results of the studies are presented in Tables 6-7, below.

Study 2526 in fasted subjects

An open-label, randomized, balanced, two-treatment, two-period, two-sequence, single-dose, crossover oral bioequivalence study of the Solifenacin Succinate/Tamsulosin Hydrochloride, modified-release tablets, 6mg/0.4mg according to Formulation Example 3 of the present invention and the marketed prior art formulation Vesomni® 6 mg/0.4 mg modified-release tablets was carried out in healthy adult human male subjects under fasting conditions.
- Pharmacokinetic data for Solifenacin in fasted subjects

Table 6.1

| **Pharmacokinetic parameter** | **Arithmetic Means (±SD)** | |
|---|---|---|
| | **Test product (Treatment A)** | **Reference product (Treatment B)** |
| $AUC_{72}$ | 365352.95 (±97572.20) | 349616.09 (±93284.53) |
| $C_{max}$ | 9728.03 (±2327.97) | 9753.02 (±2194.15) |
| $T_{max}$* | 5.50 (2.00 -7.00) | 5.00 (2.50 - 8.00) |
| **Median (Min, Max) Treatment A (Test): Solifenacin succinate/Tamsulosin hydrochloride, modified-release tablets, 6mg/0.4mg; Batch No:12118718; according to Formulation Example 3 (Manufactured by: Adamed Pharma S.A., Pilsudskiego 5, 95-200 Pabianice, Poland) Treatment B (Reference):* Vesomni® *6 mg/0,4 mg modified-release tablets; Batch No:18G27/35; (Manufactured by: Astellas Pharma Europe, B. V. Sylviusweg 62, 2333 BE Leiden, Netherlands Marketing Authorization Holder: Astellas Pharma d.o.o., Smartinska cesta 53, 1000 Ljubljana, Slovenia.)* | | |

- Phamacokinetic data for Tamsolusin in fasted subjects

Table 6.2

| **Pharmacokinetic parameter** | **Arithmetic Means (±SD)** | |
|---|---|---|
| | **Test product (Treatment A)** | **Reference product (Treatment B)** |
| $AUC_t$ | 136075.68 (±69223.76) | 129572.30 (±68982.57) |
| $AUC_{inf}$ | 142539.45 (±72830.54) | 134630.01 (±71145.91) |
| $C_{max}$ | 7114.19 (±2389.94) | 6286.22 (±2420.60) |
| $T_{max}$* | 5.00 (2.00-10.00) | 5.00 (3.00 -24.00) |
| **Median (Min, Max)*<br>*Treatment A (Test) and Treatment B (Reference) as above* | | |

- Solifenacin Bioequivalence evaluation for Solifenacin Succinate/Tamsulosin Hydrochloride, modified-release Tablets, 6mg/0.4mg prepared according to Formulation Example 3; reference: Vesomni® 6 mg/0.4 mg modified-release tablets in fasted subjects (requirements for bioequivalence: 80 % <T/R<125%)

Table 6.3

| **Pharmacokinetic parameter** | **Geometric Mean Ratio (GMR) Test / Reference** | **Confidence Intervals (CI)** | **Coefficient of variation (CV%)** |
|---|---|---|---|
| $AUC_{72}$ | 104.41 | 100.64 - 108.32 | 7.76 |
| $C_{max}$ | 99.53 | 94.79 - 104.51 | 10.31 |
| *Treatment A (Test) and Treatment B (Reference) as above* | | | |

- Tamsulosin Bioequivalence evaluation for Solifenacin Succinate/Tamsulosin Hydrochloride, modified-release tablets, 6mg/0.4mg prepared according to Formulation Example 3; reference: Vesomni® 6 mg/0.4 mg modified-release tablets in fasted subjects (requirements for bioequivalence: 80 % <T/R<125%)

Table 6.4

| Pharmacokinetic parameter | Geometric Mean Ratio (GMR) Test / Reference | Confidence Intervals (CI) | Coefficient of variation (CV%) |
|---|---|---|---|
| $AUC_t$ | 105.44 | 96.39 - 115.35 | 27.16 |
| $AUC_{inf}$ | 106.27 | 97.32 - 116.06 | 26.63 |
| $C_{max}$ | 114.54 | 106.24 - 123.49 | 22.64 |
| *Treatment A (Test) and Treatment B (Reference) as above* | | | |

Study 2527 in fed subjects

An open-label, randomized, balanced, two-treatment, two-period, two-sequence, single-dose, crossover oral bioequivalence study of the Solifenacin Succinate/Tamsulosin Hydrochloride, modified-release tablets, 6mg/0.4mg according to Formulation Example 3 of the present invention and the marketed prior art formulation Vesomni® 6 mg/0.4 mg modified-release tablets was carried out in healthy adult human male subjects under fed conditions.

- Pharmacokinetic data for Solifenacin in fed subjects

Table 7.1

| Pharmacokinetic parameter | Arithmetic Means (±SD) | |
|---|---|---|
| | Test product (Treatment A) | Reference product (Treatment B) |
| $AUC_{72}$ | 348414.92 (±77744.74) | 343853.73 (±86443.78) |
| $C_{max}$ | 8094.95 (±1652.94) | 8411.75 (±2271.44) |
| $T_{max}$* | 6.25 (2.50 -16.02) | 6.25 (2.00 -12.03) |
| **Median (Min, Max)*<br>*Treatment A (Test) and Treatment B (Reference) as above* | | |

- Pharmacokinetic data for Tamsulosin in fed subjects

Table 7.2

| Pharmacokinetic parameter | Arithmetic Means (±SD) | |
|---|---|---|
| | Test product (Treatment A) | Reference product (Treatment B) |
| $AUC_t$ | 177488.92 (±70407.72) | 180150.03 (±87748.82) |
| $AUC_{inf}$ | 182932.22 (±74682.29) | 186414.15 (±93858.59) |
| $C_{max}$ | 10981.89 (±3232.16) | 11037.64 (±3068.54) |
| $T_{max}$* | 8.00 (4.00-12.00) | 7.00 (4.00-12.00) |
| **Median (Min, Max)*<br>*Treatment A (Test) and Treatment B (Reference) as above* | | |

- Solifenacin Bioequivalence evaluation for Solifenacin Succinate/Tamsulosin Hydrochloride, modified-release tablets, 6mg/0.4mg prepared according to Formulation Example 3; reference: Vesomni® 6 mg/0.4 mg modified- release tablets in fed subjects (requirements for bioequivalence: 80 % $<T/R<$125%)

Table 7.3

| Pharmacokinetic parameter | Geometric Mean Ratio (GMR) Test / Reference | Confidence Intervals (CI) | Coeffiecient of variation (CV%) |
|---|---|---|---|
| $AUC_{72}$ | 101.54 | 98.36 - 104.83 | 7.75 |

(continued)

| Pharmacokinetic parameter | Geometric Mean Ratio (GMR) Test / Reference | Confidence Intervals (CI) | Coeffiecient of variation (CV%) |
|---|---|---|---|
| $C_{max}$ | 97.13 | 92.93 - 101.52 | 10.77 |
| *Treatment A (Test) and Treatment B (Reference) as above* | | | |

- Tamsulosin Bioequivalence evaluation for Solifenacin Succinate/Tamsulosin Hydrochloride, modified-release tablets, 6mg/0.4mg prepared according to Formulation Example 3; reference: Vesomni® 6 mg/0.4 mg modified-release tablets in fed subjects (requirements for bioequivalence: 80 % <T/R<125%)

Table 7.4

| Pharmacokinetic parameter | Geometric Mean Ratio (GMR) Test / Reference | Confidence Intervals (CI) | Coefficient of variation (CV%) |
|---|---|---|---|
| $AUC_t$ | 100.70 | 93.07 - 108.94 | 18.94 |
| $AUC_{inf}$ | 100.48 | 92.80 - 108.79 | 19.14 |
| $C_{max}$ | 98.35 | 91.58 - 105.61 | 17.12 |
| *Treatment A (Test) and Treatment B (Reference) as above* | | | |

Conclusion

As can be derived from Tables 6.1 and 6.3, above, the Solifenacin succinate/Tamsulosin hydrochloride, modified-release tablets according to Formulation Example 3 of the present invention, demonstrated a comparable extent and rate of absorption of solifenacin to the reference product Vesomni® 6 mg/0,4 mg modified-release tablets with a Test/Reference (T/R) geometric mean ratios (GMR) of approximately 104% and 100% for $AUC_{72}$ and $C_{max}$, respectively, with the corresponding 90% confidence intervals (CIs) falling within the EMA-acceptance range 80.00% - 125.00%.

Moreover, the Solifenacin succinate/Tamsulosin hydrochloride, modified-release tablets, according to Formulation Example 3 of the present invention also demonstrated a comparable extent of absorption of tamsulosin to the Reference product Vesomni® 6 mg/0,4 mg modified release tablets with a Test/Reference (T/R) geometric mean ratios (GMR) of approximately 105%, 106% and 115% for $AUC_{t.}$, $AUC_{inf}$ and $C_{max}$, respectively and with the corresponding 90% confidence intervals (CIs) falling within the EMA-acceptance range 80.00% - 125.00%, see Tables 6.2 and 6.4, above.

Thus, bioequivalence between the Solifenacin succinate/Tamsulosin hydrochloride, modified-release tablets according to Formulation Example 3 of the present invention and the reference product Vesomni® 6 mg/0,4 mg modified-release tablets has been established for solifenacin and tamsulosin in healthy, adult, human, male subjects under fasting conditions.

As can be derived from Tables 7.1 and 7.3, above, the Solifenacin succinate/Tamsulosin hydrochloride, modified-release tablets according to Formulation Example 3 of the present invention, demonstrated a comparable extent and rate of absorption of solifenacin to the reference product Vesomni® 6 mg/0,4 mg modified-release tablets with a Test/Reference (T/R) geometric mean ratios (GMR) of approximately 102% and 97% for $AUC_{72}$ and $C_{max}$, respectively and with the corresponding 90% confidence intervals (CIs) falling within the EMA-acceptance range 80.00% - 125.00%.

Moreover, the Solifenacin succinate/Tamsulosin hydrochloride, modified-release tablets, according to Formulation Example 3 of the present invention also demonstrated a comparable extent of absorption of tamsulosin to the reference product Vesomni® 6 mg/0,4 mg modified release tablets with a Test/Reference (T/R) geometric mean ratios (GMR) of approximately 101%, 100% and 98% for $AUC_t$, $AUC_{inf}$ and $C_{max}$, respectively. The corresponding 90% confidence intervals (CIs) for AUCt and $C_{max}$ fall within the EMA-acceptance range 80.00% - 125.00%, see Tables 7.2 and 7.4, above.

Thus, bioequivalence between the Solifenacin succinate/Tamsulosin hydrochloride, modified-release tablets according to Formulation Example 3 of the present invention and the Reference product Vesomni® 6 mg/0,4 mg modified release tablets was also established for solifenacin and tamsulosin in healthy, adult, human, male subjects under fed conditions.

Moreover, as can be derived from Table 6.1, above, the formulation according to the present invention provided a higher $AUC_{72}$ for solifenacin in fasted subjects, also the range of the observed $t_{max}$ for solifenacin in fasted subjects exhibited lower minimum and maximum limits. These data are in accordance with the dissolution data observed (see Table 4, above), evidencing that a faster and more complete dissolution of solifenacin succinate from the formulation of the

present invention leads to a faster and higher systemic availability of solifenacin in fasted subjects.

Thus, while it has been demonstrated that the formulation according to the present invention are bioequivalent to the marketed prior art product Vesomni® 6 mg/0,4 mg modified release tablets, they provide a faster and more complete release of solifenacin resulting in a higher $AUC_{72}$ and smaller $t_{max}$ in fasted subjects, which can be desirable/advantageous in certain patient populations.

Comparative test with prior art formulations according to EP 3 697 392

Qualitative and quantitative compositions of solifenacin and tamsulosin layers, respectively, of the multilayer tablet as disclosed in Examples 1 and 2 of EP 3 697 392 were prepared and tableted to the bi-layered tablet according to the teaching of this document.

Problems were, however, encountered in preparing a satisfactory solifenacin layer in accordance with the teaching of Example 1 of EP 3 697 392.

Thus, although the API (solifenacin) and the diluent were sieved (in an attempt to prevent agglomeration) prior to the high shear granulation in the presence of ethanol, the granulation resulted in irregular small agglomerates of different size. Presumably due to the lack of a binder, no satisfactory granules could be obtained. The subsequent blending of Aerosil, crosscarmellose and finally with magnesium stearate resulted in a non-coherent mass with poor flow properties. As a result, high variation of the tablet mass was observed. Moreover, the coating of the tablets was problematic due to tablet weight variation and due problems with compression resulting in surface defects.

The physical properties of the tablets in accordance with EP 3 697 392 were assessed, including the tensile strength, friability and uniformity of mass.

As set out in Table 8 below, the bilayered formulations of EP 3 697 392 were unsatisfactory particularly in relation with the uniformity of mass of the dosage unit.

Thus, unlike the formulations of the invention which exhibited a dosage uniformity in accordance with the pharmacopeial requirements, the degree of uniformity observed with the prior art formulation was significantly below the acceptable standard. This is of considerable significance as consistency of dosage units is mandatory to ensure that each unit of a batch has a drug content within a narrow range around the specified dose. The formulation of the invention, by virtue of the specific excipients used thus has unexpected favourable properties compared to the prior art formulations.

Table 8

| | Acceptance Limit | Example 3 according to the invention | EP 3 697 392 Example 1 |
|---|---|---|---|
| | | Fujicalin/Prosolv HD90 | Avicel DG, Silicon Dioxide |
| Bulk density | 0.35 - 0.55 | 0.48 | 0.41 |
| Tapped density | 0.50 - 0,65 | 0.6 | 0.6 |
| Carr index | <22 | 20 | 32 |
| Hausner ratio | <1.30 | 1.25 | 1.6 |
| Sieve analysis | d50 >80μm | d10=25μm<br>d50=109μm<br>α90=199μm | d10=10μm<br>d50=57μm<br>d90=115μm |
| deviation %, average weight of whole tablet | Pharmacopeial criteria for uniformity of mass of single-dose: ±5.0% (min 18 measurements) and ±10.0 (maximum 2 measurements) for the whole tablet | ±1.5% for all tablets | ±20.0% for 8 of 20 tablets |
| coating defects - edge chipping, surface erosion | visually inspected integrity and quality of the tablet | not observed | uneven surface of solifenacin layer cup |

Industrial Applicability

The present invention provides a pharmaceutical composition for oral administration comprising a modified release layer comprising tamsulosin or a pharmaceutically acceptable salt thereof, and an immediate release layer

comprising solifenacin or a pharmaceutically acceptable salt thereof. The pharmaceutical composition of the present invention exhibits release profiles equivalent to those of the current single drug formulations, and thus provides both active ingredients in a single formulation which elicits pharmacological effects equivalent to those of the current single drug formulations and provides a bioequivalent yet improved formulation for the combination formulation currently on the market.

While the present invention is explained herein with reference to particular embodiments, modifications and improvements obvious to those skilled in the art are included in the scope of the present invention.

The contents of all documents (patent documents and other references) cited in the present application are incorporated herein in their entirety by reference.

The invention is summarized by the following items:

1. A tablet for oral administration comprising

(1) a first modified release layer comprising tamsulosin or a pharmaceutically acceptable salt thereof, a matrix forming polymer, and optionally one or more excipients selected from a hydrophilic additive, a diluent, a glidant and a lubricant;

(2) a second immediate release layer comprising crystalline solifenacin succinate and a combination of silicified microcrystalline cellulose (SMCC) and Dicalcium phosphate (DCP) or anhydrous Dicalcium phosphate (DCPA) at a ratio of 2.0-3.8 : 0.7-1.3, preferably at a ratio of 3:1

and optionally one or more excipient selected from a disintegrant, a binder and a lubricant,

wherein the tablet is optionally film coated.

2. Tablet according to item 1, which is a bi-layered tablet.
3. Tablet according to items 1 or 2, wherein the first modified release layer comprises tamsulosin HCl.
4. Tablet according any one of items 1-3, wherein the matrix forming polymer is Macrogol 7 000 000.
5. Tablet according to any one of items 1-4, wherein the first modified release layer comprises microcrystalline cellulose type 200, colloidal anhydrous silica and magnesium stearate.
6. Tablet according to any one of items 1-5, wherein the second immediate release layer comprises low-substituted hydroxypropyl cellulose and magnesium stearate.
7. Tablet according to any one of items 1-6 having the following composition

| **Modified release layer** | % of total tablet mass | **Corresponding to % of mass of modified release layer** |
|---|---|---|
| Tamsulosin hydrochloride | 0.114 | 0.16 |
| Macrogol 7 000 000 | 42-67.86 | 60-95 |
| Cellulose, Microcrystalline type 200 | 3.71-25 | 5-35 |
| Silica, Colloidal Anhydrous | 0.071-0.71 | 0.1-1.0 |
| Magnesium stearate | 0.071-2.14 | 0.1-3.0 |
| **Immediate release layer** | | **Corresponding to % of mass of immediate release layer** |
| Solifenacin succinate | 1.714 | |
| DCP or DCPA | 5-12 | 15-36 |
| Silicified Microcrystalline Cellulose | 8.57-21.14 | 30-95 |
| Low-Substituted Hydroxypropyl Cellulose | 0.28-1.142 | 1-4 |
| Magnesium stearate | 0.0285-0.857 | 0.1-3.0 |
| Total mass of uncoated tablet: | 100.00 | |
| **Film coating (optional)** | | |
| Opadry® complete Film coating system 03F45072 RED | 3.00 | |
| Total mass of film-coated tablet | 103.00 | |

8. Tablet according to any one of items 1-7, having the following composition

| **Modified release layer** | % of total tablet mass | Corresponding to % of mass of modified release layer |
|---|---|---|
| Tamsulosin hydrochloride | 0.114 | 0.16 |
| Macrogol 7 000 000 | 57.143 | 80 |
| Cellulose, Microcrystalline type 200 | 13.429 | 18.80 |
| Silica, Colloidal Anhydrous | 0.371 | 0.52 |
| Magnesium stearate | 0.371 | 0.52 |
| **Immediate release layer** | | Corresponding % of mass of immediate release layer |
| Solifenacin succinate | 1.714 | 6% |
| Dibasic Calcium Phosphate Anhydrous | 6.571 | 23% |
| Silicified Microcrystalline Cellulose | 19.571 | 68.5% |
| Low-Substituted Hydroxypropyl Cellulose | 0.571 | 2% |
| Magnesium stearate | 0.143 | 0.5% |
| Total mass of uncoated tablet: | 100.00 | |
| **Film coating (optional)** | | |
| Opadry® complete Film coating system 03F45072 RED | 3.00 | |
| Total mass of film-coated tablet: | 103.00 | |

9. Tablet of any one of items 1-8 having the following composition

| | mg/tablet |
|---|---|
| **Modified release layer** | |
| Tamsulosin hydrochloride | 0.4 |
| Macrogol 7 000 000 | 200.0 |
| Cellulose, Microcrystalline type 200 | 47.0 |
| Silica, Colloidal Anhydrous | 1.3 |
| Magnesium stearate | 1.3 |
| **Immediate release layer** | |
| Solifenacin succinate | 6.0 |
| Dibasic Calcium Phosphate Anhydrous | 23.0 |
| Silicified Microcrystalline Cellulose | 68.5 |
| Low-Substituted Hydroxypropyl Cellulose | 2.0 |
| Magnesium stearate | 0.5 |
| Total mass of uncoated tablet: | 350.0 |
| **Film coating (optional)** | |
| Opadry® complete Film coating system 03F45072 RED | 10.5 |
| Total mass of film-coated tablet: | 360.5 |

10. Tablet according to any one of items 1-9, wherein the SMCC has a BET of about 6 $m^2/g$ and a mean particle size of about 125 μm.

11. Tablet according to any one of items 1-10, wherein the DCP or DCPA has a BET of greater than 35 $m^2/g$, preferably of about 40 $m^2/g$ and mean particle size of 115 -120 µm.
12. Tablet according to any one of items 1-11, wherein the SMCC has a BET of about 6 $m^2/g$ and a mean particle size of about 125 µm and the DCP or DCPA has a BET of greater than 35 $m^2/g$, preferably about 40 $m^2/g$ and a mean particle size of about 115-120 µm.
13. Tablet according to any one of items 1-12, wherein at least 94.5% solifenacin is dissolved after 10 min in accordance with a dissolution test in 0.1 M HCl (pH 1.2), 500 ml, 100 RPM, temperature 37 °C ± 0.5 °C, Apparatus type: 1 (basket, according to European Pharmacopoeia 8$^{th}$ edition).
14. Tablet according to any one of items 1-13 wherein at least 95.3% solifenacin is dissolved after 15 min in accordance with the dissolution test in 0.1 M HCl (pH 1.2), 500 ml, 100 RPM, temperature 37 °C ± 0.5 °C, Apparatus type: 1 (basket, according to European Pharmacopoeia 8$^{th}$ edition).
15. Tablet according to any one of items 1-14, wherein at least 96.9% solifenacin is dissolved after 30 min in accordance with the dissolution test in 0.1 M HCl (pH 1.2), 500 ml, 100 RPM, temperature 37 °C ± 0.5 °C, Apparatus type: 1 (basket, according to European Pharmacopoeia 8$^{th}$ edition).
16. Tablet according to any one of items 1-15, wherein at least 18.7% tamsulosin is dissolved after 2 h in accordance with the dissolution test in phosphate buffer pH 6.8, 500 ml, 50 RPM, temperature 37 °C ± 0.5 °C, Apparatus type: 2 (paddles with sinkers, according to Japan Pharmacopoeia 15$^{th}$ edition).
17. Tablet according to any one of items 1-16, wherein at least 48.9 tamsulosin is dissolved after 8 h in accordance with the dissolution test in phosphate buffer pH 6.8, 500 ml, 50 RPM, temperature 37 °C ± 0.5 °C, Apparatus type: 2 (paddles with sinkers, according to Japan Pharmacopoeia 15$^{th}$ edition).
18. Tablet according to any one of items 1-17, wherein at least 87.7% of tamsulosin is dissolved after 21 h in accordance with the dissolution test in accordance with the dissolution test in phosphate buffer pH 6.8, 500 ml, 50 RPM, temperature 37 °C ± 0.5 °C, Apparatus type: 2 (paddles with sinkers, according to Japan Pharmacopoeia 15$^{th}$ edition).
19. Tablet according to any one of items 1-18, wherein solifenacin succinate is crystalline solifenacin succinate polymorphic form I.
20. Process for preparing a tablet according any one of items 1-19, comprising the step of

(1) preparing granulate for the immediate release layer comprising solifenacin succinate by

(a) mixing solifenacin succinate, SMCC and the binder/disintegrant or disintegrant in a granulator, preferably in a high shear granulator;
(b) spraying the blend obtained in (a) with granulation solvent and granulate;
(c) passing the wet granulate obtained in step (b) through 5 mm sieve and drying the granulate in a fluid bed dryer;
(d) passing the dried granulate through 1 mm mesh;
(e) stepwise adding the granulate external phase - DCP or DCPA - and blending thoroughly and
(f) adding lubricant through 0.5 mm sieve to the blend obtained in step (e); and blending thoroughly;
or

(1a) preparing granulate for the immediate release layer comprising solifenacin succinate by

(g) mixing solifenacin succinate and the water-insoluble excipients DCP or DCPA and SMCC in a granulator, preferably in a high shear granulator;
(h) spraying the blend obtained in (g) with granulation solvent and granulate;
(i) passing the wet granulate obtained in step (h) through 5 mm sieve and drying the granulate in a fluid bed dryer;
(j) passing the dried granulate through 1 mm mesh;
(k) stepwise adding the granulate external phase (binder/disintegrant, disintegrant) and blending thoroughly and
(l) adding the lubricant through 0.5 mm sieve to the blend obtained in step (k); and blending thoroughly.

21. Process for preparing a tablet according to any one of items 1 to 19 comprising the following steps:

(1) preparing granulate for the immediate release layer comprising solifenacin succinate by

(a) mixing solifenacin succinate, SMCC and the binder/disintegrant, disintegrant in a granulator, preferably in a high shear granulator;
(b) spraying the blend obtained in (a) with granulation solvent and granulate;
(c) passing the wet granulate obtained in step (b) through 5 mm sieve and drying the granulate in a fluid bed dryer;
(d) passing the dried granulate through 1 mm mesh;
(e) stepwise adding the granulate external phase - DCP or DCPA - and blending thoroughly and

(f) adding the lubricant through 0.5 mm sieve to the blend obtained in step (e); and blending thoroughly; or

(1a) preparing granulate for the immediate release layer comprising solifenacin succinate by

(g) mixing solifenacin succinate and the water-insoluble excipients DCP or DCPA and SMCC in a granulator, preferably in a high shear granulator;
(h) spraying the blend obtained in (g) with granulation solvent and granulate;
(i) passing the wet granulate obtained in step (h) through 5 mm sieve and drying the granulate in a fluid bed dryer;
(j) passing the dried granulate through 1 mm mesh;
(k) stepwise adding the granulate external phase -binder/disintegrant or disintegrant and blending thoroughly and
(l) adding lubricant through 0.5 mm sieve to the blend obtained in step (k); and blending thoroughly;

(2) preparing granulate for the modified release layer comprising tamsulosin HCl by

(m) dissolving tamsulosin HCl in the granulation solvent thereby obtaining a granulation liquid;
(n) spraying the solution obtained in step (m) onto the matrix forming polymer Macrogol 7 000 000 using a fluid bed granulator to obtain a granulate;
(o) sieving the obtained (dry) granulate through a 1 mm mesh;
(p) stepwise adding the granulate external phase (diluent and glidant), and blending thoroughly and
(q) adding the lubricant through 0.5 mm sieve to the blend obtained in step (p) and blending thoroughly;

(3) compressing granulates obtained in steps (1) or (1a) and (2) into bilayer tablets using a bilayer tableting machine and

(4) optionally film coating the tablet cores obtained in step (3) to a weight gain of about 3%.

22. Process according to item 21, comprising steps (1), (2), (3) and (4).
23. Process according to any one of items 20 to 22, wherein solifienacin succinate is crystalline solifenacin succinate polymorphic form I.
24. Tablet obtained by a process according to any one of items 19 to 22, wherein solifenacin succinate is crystalline solifenacin succinate polymorphic form I.

## Claims

1. A tablet for oral administration comprising

(1) a first modified release layer comprising tamsulosin or a pharmaceutically acceptable salt thereof, a matrix forming polymer, and optionally one or more excipients selected from a hydrophilic additive, a diluent, a glidant and a lubricant;
(2) a second immediate release layer comprising crystalline solifenacin succinate and a combination of silicified microcrystalline cellulose (SMCC) and Dicalcium phosphate (DCP) or anhydrous Dicalcium phosphate (DCPA) at a ratio of 2.0-3.8 : 0.7-1.3, preferably at a ratio of 3:1
and optionally one or more excipients selected from a disintegrant, a binder and a lubricant,
wherein the tablet is optionally film coated.

2. Tablet according to claim 1, which is a bi-layered tablet.

3. Tablet according to claims 1 or 2, wherein the first modified release layer comprises tamsulosin HCl.

4. Tablet according any one of claims 1-3, wherein the matrix forming polymer is Macrogol 7 000 000.

5. Tablet according to any one of claims 1-4, wherein the first modified release layer comprises microcrystalline cellulose type 200, colloidal anhydrous silica and magnesium stearate.

6. Tablet according to any one of claims 1-5, wherein the second immediate release layer comprises low-substituted hydroxypropyl cellulose and magnesium stearate.

**7.** Tablet according to any one of claims 1-6 having the following composition:

| **Modified release layer** | % of total tablet mass | **Corresponding to % of mass of modified release layer** |
|---|---|---|
| Tamsulosin hydrochloride | 0.114 | 0.16 |
| Macrogol 7 000 000 | 42-67.86 | 60-95 |
| Cellulose, Microcrystalline type 200 | 3.71-25 | 5-35 |
| Silica, Colloidal Anhydrous | 0.071-0.71 | 0.1-1.0 |
| Magnesium stearate | 0.071-2.14 | 0.1-3.0 |
| **Immediate release layer** | | **Corresponding to % of mass of immediate release layer** |
| Solifenacin succinate | 1.714 | |
| DCP or DCPA | 5-12 | 15-36 |
| Silicified Microcrystalline Cellulose | 8.57-21.14 | 30-95 |
| Low-Substituted Hydroxypropyl Cellulose | 0.28-1.142 | 1-4 |
| Magnesium stearate | 0.0285-0.857 | 0.1-3.0 |
| Total mass of uncoated tablet: | 100.00 | |
| **Film coating (optional)** | | |
| Opadry® complete Film coating system 03F45072 RED | 3.00 | |
| Total mass of film-coated tablet | 103.00 | |

or

| **Modified release layer** | % of total tablet mass | Corresponding to % of mass of modified release layer |
|---|---|---|
| Tamsulosin hydrochloride | 0.114 | 0.16 |
| Macrogol 7 000 000 | 57.143 | 80 |
| Cellulose, Microcrystalline type 200 | 13.429 | 18.80 |
| Silica, Colloidal Anhydrous | 0.371 | 0.52 |
| Magnesium stearate | 0.371 | 0.52 |
| **Immediate release layer** | | Corresponding % of mass of immediate release layer |
| Solifenacin succinate | 1.714 | 6% |
| Dibasic Calcium Phosphate Anhydrous | 6.571 | 23% |
| Silicified Microcrystalline Cellulose | 19.571 | 68.5% |
| Low-Substituted Hydroxypropyl Cellulose | 0.571 | 2% |
| Magnesium stearate | 0.143 | 0.5% |
| Total mass of uncoated tablet: | 100.00 | |
| **Film coating (optional)** | | |
| Opadry® complete Film coating system 03F45072 RED | 3.00 | |
| Total mass of film-coated tablet: | 103.00 | |

or

| | mg/tablet |
|---|---|
| **Modified release layer** | |
| Tamsulosin hydrochloride | 0.4 |
| Macrogol 7 000 000 | 200.0 |
| Cellulose, Microcrystalline type 200 | 47.0 |
| Silica, Colloidal Anhydrous | 1.3 |
| Magnesium stearate | 1.3 |
| **Immediate release layer** | |
| Solifenacin succinate | 6.0 |
| Dibasic Calcium Phosphate Anhydrous | 23.0 |
| Silicified Microcrystalline Cellulose | 68.5 |
| Low-Substituted Hydroxypropyl Cellulose | 2.0 |
| Magnesium stearate | 0.5 |
| Total mass of uncoated tablet: | 350.0 |
| **Film coating (optional)** | |
| Opadry® complete Film coating system 03F45072 RED | 10.5 |
| Total mass of film-coated tablet: | 360.5 |

**8.** Tablet according to any one of claims 1-7, wherein the SMCC has a BET of about 6 $m^2/g$ and a mean particle size of about 125 μm and/or
wherein the DCP or DCPA has a BET of greater than 35 $m^2/g$, preferably of about 40 $m^2/g$ and a mean particle size of 115 -120 μm.

**9.** Tablet according to any one of claims 1-8, wherein at least 94.5% solifenacin is dissolved after 10 min in accordance with a dissolution test in 0.1 M HCl (pH 1.2), 500 ml, 100 RPM, temperature 37 °C ± 0.5 °C, Apparatus type: 1 (basket, according to European Pharmacopoeia 8th edition); or

wherein at least 95.3% solifenacin is dissolved after 15 min in accordance with the dissolution test in 0.1 M HCl (pH 1.2), 500 ml, 100 RPM, temperature 37 °C ± 0.5 °C, Apparatus type: 1 (basket, according to European Pharmacopoeia 8th edition); or
wherein at least 96.9% solifenacin is dissolved after 30 min in accordance with the dissolution test in 0.1 M HCl (pH 1.2), 500 ml, 100 RPM, temperature 37 °C ± 0.5 °C, Apparatus type: 1 (basket, according to European Pharmacopoeia 8th edition).

**10.** Tablet according to any one of claims 1-9, wherein at least 18.7% tamsulosin is dissolved after 2 h in accordance with the dissolution test in phosphate buffer pH 6.8, 500 ml, 50 RPM, temperature 37 °C ± 0.5 °C, Apparatus type: 2 (paddles with sinkers, according to Japan Pharmacopoeia 15th edition) or

wherein at least 48.9% tamsulosin is dissolved after 8 h in accordance with the dissolution test in phosphate buffer pH 6.8, 500 ml, 50 RPM, temperature 37 °C ± 0.5 °C, Apparatus type: 2 (paddles with sinkers, according to Japan Pharmacopoeia 15th edition) or
wherein at least 87.7% of tamsulosin is dissolved after 21 h in accordance with the dissolution test in phosphate buffer pH 6.8, 500 ml, 50 RPM, temperature 37 °C ± 0.5 °C, Apparatus type: 2 (paddles with sinkers, according to Japan Pharmacopoeia 15th edition).

**11.** Tablet according to any one of claims 1-10, wherein solifenacin succinate is crystalline solifenacin succinate polymorphic form I.

**12.** Process for preparing a tablet according any one of claims 1-11, comprising the steps of

(1) preparing granulate for the immediate release layer comprising solifenacin succinate by

(a) mixing solifenacin succinate, SMCC and the binder/disintegrant or disintegrant in a granulator, preferably in a high shear granulator;
(b) spraying the blend obtained in step (a) with granulation solvent and granulate;
(c) passing the wet granulate obtained in step (b) through 5 mm sieve and drying the granulate in a fluid bed dryer;
(d) passing the dried granulate through 1 mm mesh;
(e) stepwise adding the granulate external phase - DCP or DCPA - and blending thoroughly and
(f) adding lubricant through 0.5 mm sieve to the blend obtained in step (e); and blending thoroughly;
or

(1a) preparing granulate for the immediate release layer comprising solifenacin succinate by

(g) mixing solifenacin succinate and the water-insoluble excipients DCP or DCPA and SMCC in a granulator, preferably in a high shear granulator;
(h) spraying the blend obtained in step (g) with granulation solvent and granulate;
(i) passing the wet granulate obtained in step (h) through 5 mm sieve and drying the granulate in a fluid bed dryer;
(j) passing the dried granulate through 1 mm mesh;
(k) stepwise adding the granulate external phase (binder/disintegrant, disintegrant) and blending thoroughly and
(l) adding the lubricant through 0.5 mm sieve to the blend obtained in step (k); and blending thoroughly.

**13.** Process for preparing a tablet according to any one of claims 1 to 11 comprising the following steps:

(1) preparing granulate for the immediate release layer comprising solifenacin succinate by

(a) mixing solifenacin succinate, SMCC and the binder/disintegrant, disintegrant in a granulator, preferably in a high shear granulator;
(b) spraying the blend obtained in step (a) with granulation solvent and granulate;
(c) passing the wet granulate obtained in step (b) through 5 mm sieve and drying the granulate in a fluid bed dryer;
(d) passing the dried granulate through 1 mm mesh;
(e) stepwise adding the granulate external phase - DCP or DCPA - and blending thoroughly; and
(f) adding the lubricant through 0.5 mm sieve to the blend obtained in step (e); and blending thoroughly;
or

(1a) preparing granulate for the immediate release layer comprising solifenacin succinate by

(g) mixing solifenacin succinate and the water-insoluble excipients DCP or DCPA and SMCC in a granulator, preferably in a high shear granulator;
(h) spraying the blend obtained in step (g) with granulation solvent and granulate;
(i) passing the wet granulate obtained in step (h) through 5 mm sieve and drying the granulate in a fluid bed dryer;
(j) passing the dried granulate through 1 mm mesh;
(k) stepwise adding the granulate external phase - binder/disintegrant or disintegrant - and blending thoroughly and
(l) adding lubricant through 0.5 mm sieve to the blend obtained in step (k); and blending thoroughly;

(2) preparing granulate for the modified release layer comprising tamsulosin HCl by

(m) dissolving tamsulosin HCl in the granulation solvent thereby obtaining a granulation liquid;
(n) spraying the solution obtained in step (m) onto the matrix forming polymer Macrogol 7 000 000 using a fluid bed granulator to obtain a granulate;
(o) sieving the obtained (dry) granulate through a 1 mm mesh;

(p) stepwise adding the granulate external phase (diluent and glidant), and blending thoroughly; and
(q) adding the lubricant through 0.5 mm sieve to the blend obtained in step (p) and blending thoroughly;

(3) compressing granulates obtained in steps (1) or (1a) and (2) into bilayer tablets using a bilayer tableting machine and
(4) optionally film coating the tablet cores obtained in step (3) to a weight gain of about 3%;

preferably comprising steps (1), (2), (3) and (4).

**14.** Process according to claim 12 or 13, wherein solifienacin succinate is crystalline solifenacin succinate polymorphic form I.

**15.** Tablet obtained by a process according to claim 12 or 13, wherein solifenacin succinate is crystalline solifenacin succinate polymorphic form I.

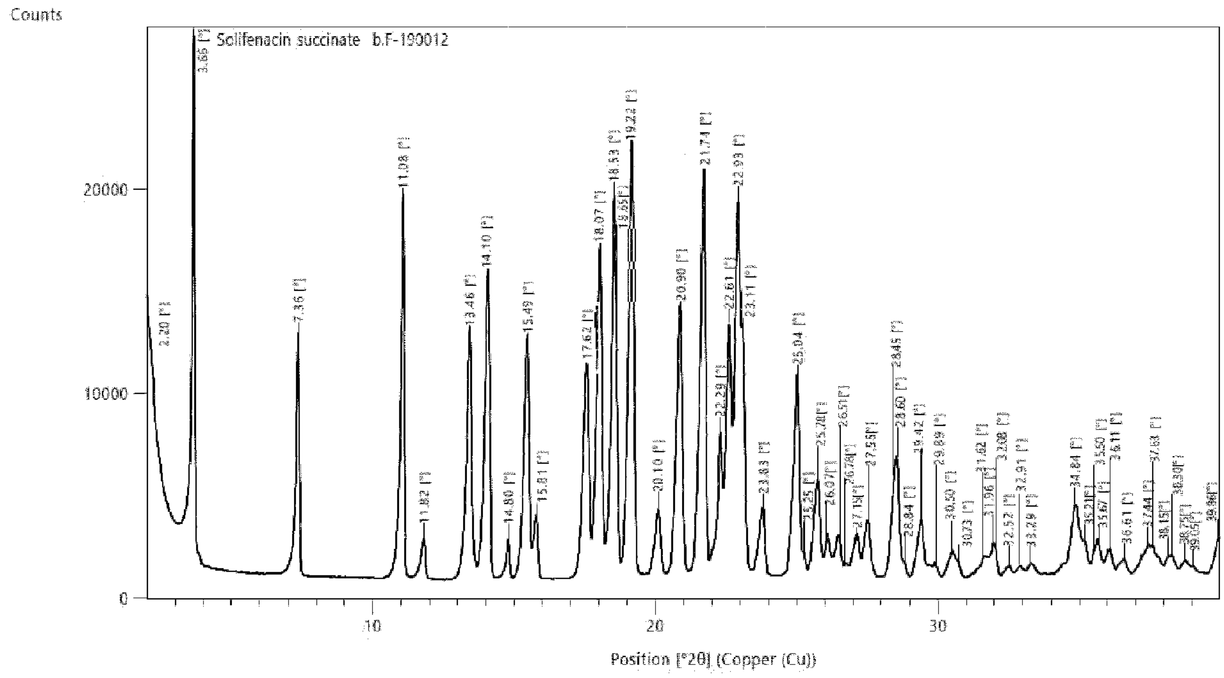


**Fig. 1**

XRD pattern of the API crystalline solifenacin succinate polymorphic form I

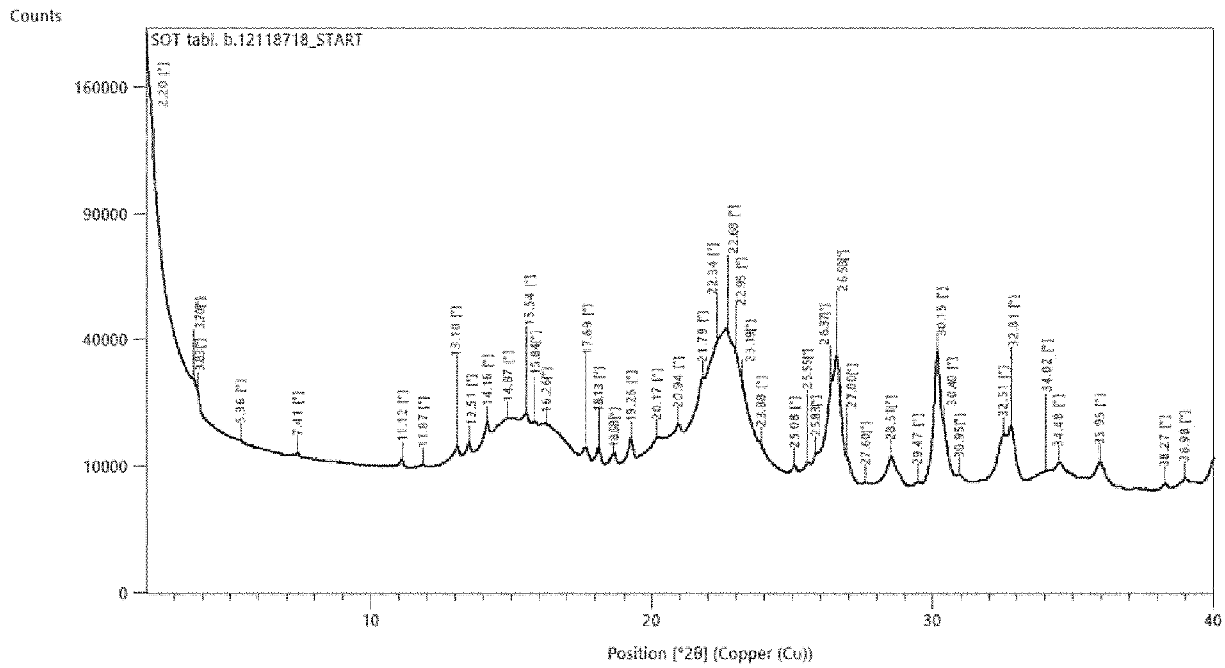


**Fig. 2**

XRD pattern of solifenacin succinate in the solifenacin layer of the tablet formulation at t=0

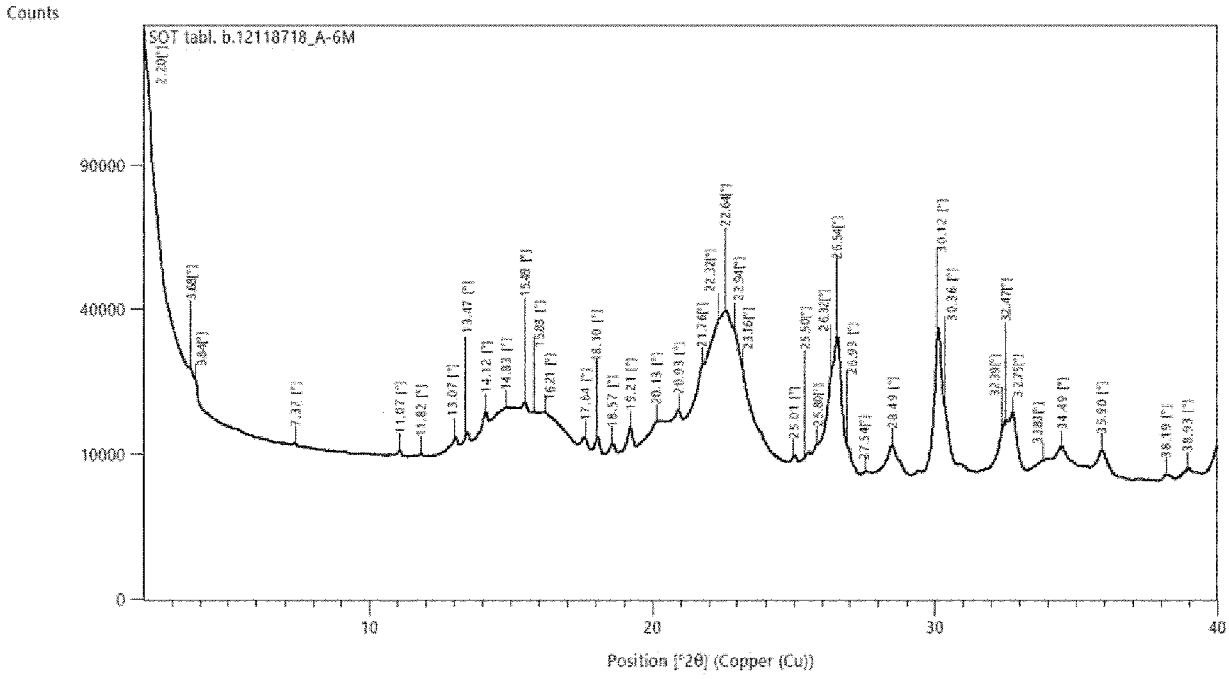


**Fig. 3**

XRD pattern of solifenacin succinate in the solifenacin layer of the table formulation after 6 months at 25°C/60% RH

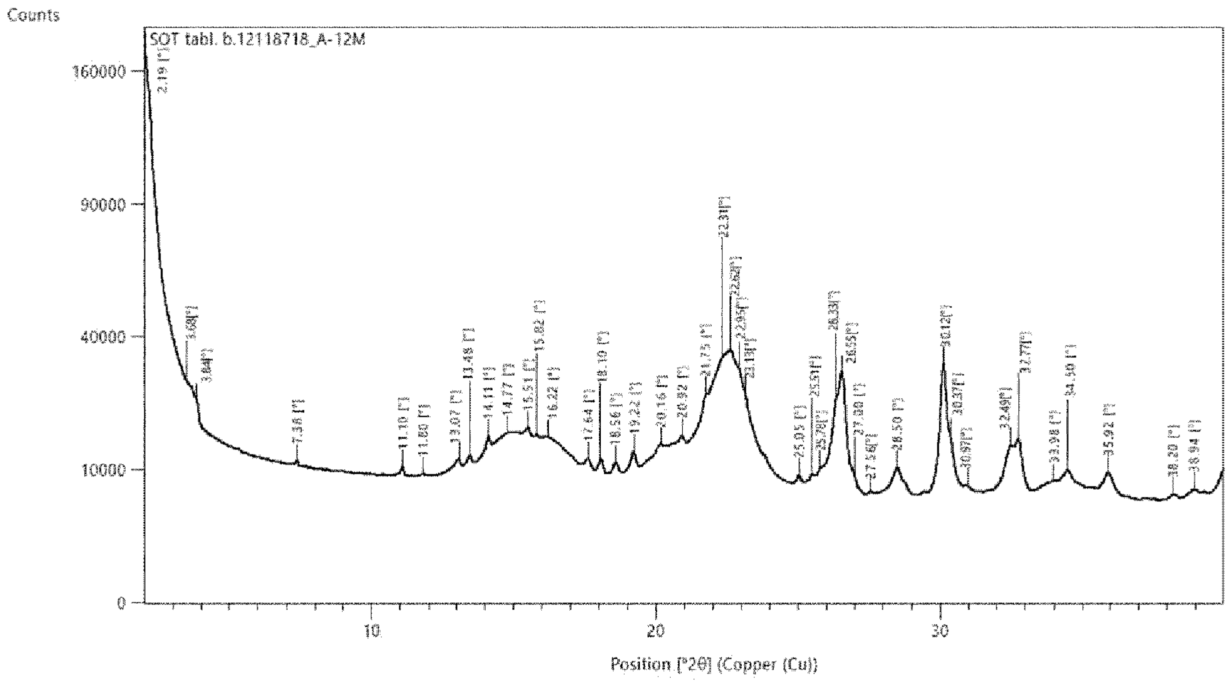


**Fig. 4**

XRD pattern of solifenacin succinate in the solifenacin layer of the tablet formulation after 12 months at 25°C/60 % RH

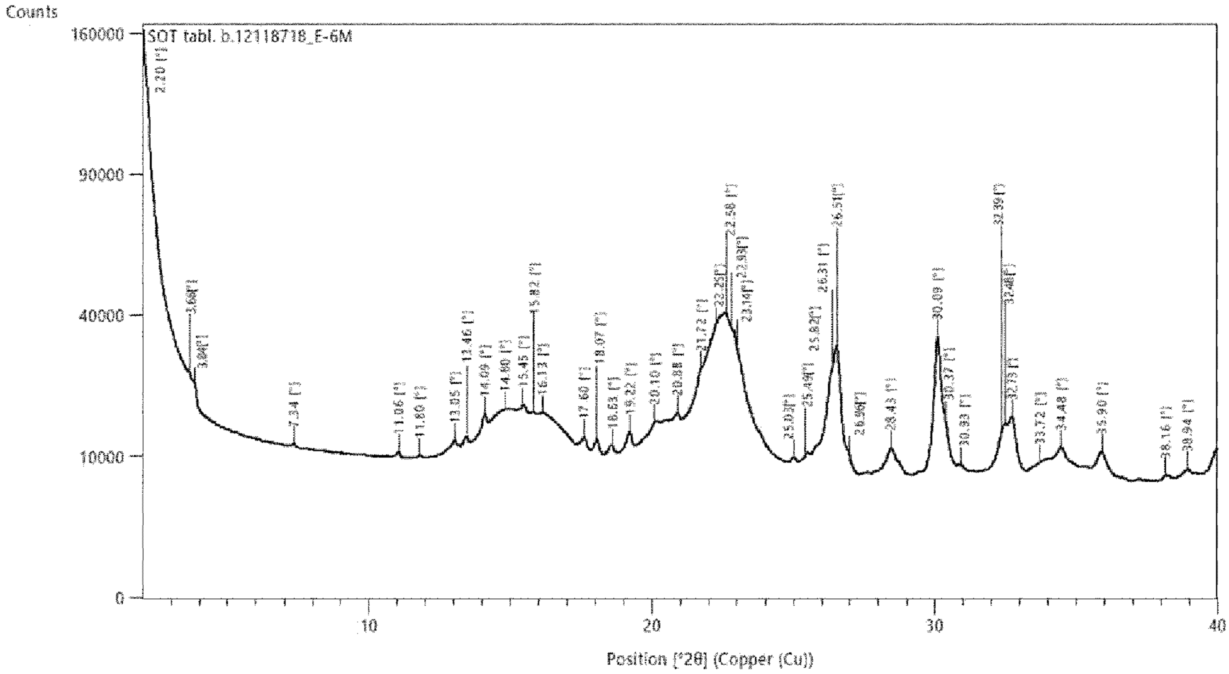


**Fig. 5**

XRD pattern of solifenacin succinate in the solifenacin layer of the tablet formulation after 6 months at 40°C/75% RH

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 661045 A
- EP 1523994 A
- EP 1832288 A
- EP 1852117 A
- EP 2178507 A
- EP 2146693 A
- EP 2394648 A
- EP 3697392 A
- US 5486365 A
- WO 2005105795 A
- WO 2008013851 A


### Non-patent literature cited in the description


- Pharmaceutical Powder Compaction Technology. Marcel Dekker, 1996
- **HECKEL RW**. Density-Pressure Relationship in Powder Compaction. *Trans Met Soc AIME*, 1961, vol. 22, 671-675
- **S. PATEL et al.** *International Journal of Pharmaceutics*, 2010, vol. 389, 66-73
- **FAWCETT, T.G.** ; **GATES-RECTOR, S.** ; **GINDHART, A.M.** ; **ROST, M.** ; **KABEKKODU, S.N.** ; **BLANTON, J.R.** ; **BLANTON, T.N.** A practical guide to pharmaceutical analyses using X-ray powder diffraction. *Powder Diffr.*, 2019, vol. 34 (2), 164-183
- Guideline on the Investigation of Bioequivalence. *CPMP/EWP/QWP/1401/98 Rev. 1/Corr***